# EUROPÄISCHE PATENTSCHRIFT

(11) **EP 3 860 540 B1**
(45) Veröffentlichungstag und Bekanntmachung des Hinweises auf die Patenterteilung: **04.11.2026**
(21) Anmeldenummer: 19782942.7
(22) Anmeldetag: 26.09.2019
(51) Int. Cl.: A61F 13/56, A61F 13/84

(54) **ABSORBIERENDE INKONTINENZWEGWERFWINDEL MIT KENNZEICHNUNGSMITTEL**
ABSORBENT DISPOSABLE INCONTINENCE DIAPER HAVING IDENTIFICATION MEANS
COUCHE ABSORBANTE JETABLE POUR INCONTINENCE DOTÉE DE MOYENS D'IDENTIFICATION

(30) Priorität: 01.10.2018 DE 102018124264
(43) Veröffentlichungstag der Anmeldung: 11.08.2021
(73) Patentinhaber: Paul Hartmann AG, 89522 Heidenheim (DE)
(72) Erfinder: DANNENMANN, Petra, 89542 Herbrechtingen (DE); LIMAM, Deniz, 892321 Neu-Ulm (DE); BUCH, Tamara, 89077 Ulm (DE); EILERS, Jörg, 89179 Beimerstetten (DE); SCHMIDT, Ann-Cathrin, 06667 Weißenfels (DE); BEYRLE, Andreas, 89564 Nattheim (DE); KESSELMEIER, Rüdiger, 89233 Burlafingen (DE)
(74) Vertreter: Paul Hartmann AG Patents & Licensing
(86) Internationale Anmeldenummer: PCT/EP2019/076045
(87) Internationale Veröffentlichungsnummer: WO 2020/069967

(56) Entgegenhaltungen:
- WO-A1-2017/114721
- WO-A2-2009/117492

## Beschreibung

Die vorliegende Erfindung betrifft eine absorbierende Inkontinenzwegwerfwindel, mit einer Längsrichtung und einer Querrichtung, mit einer Längsmittelachse und einer Quermittelachse, mit einer Innenseite und einer Außenseite, mit einem einen Saugkörper aufweisenden Hauptteil mit einem hinteren Hüftrand und einem vorderen Hüftrand, und umfassend einen Rückenbereich, einen Vorderbereich, und einen in Längsrichtung dazwischen liegenden, zwischen den Beinen eines Benutzers zu liegen kommenden Schrittbereich, und mit beidseits im Rückenbereich vorgesehenen hinteren Seitenabschnitte, welche sich in Querrichtung zumindest über seitliche mittlere Längsränder des Schrittbereichs hinaus erstrecken und jeweils wenigstens ein Verschlussmittel aufweisen, das an dem Vorderbereich lösbar festlegbar ist und dadurch der Vorderbereich und der Rückenbereich miteinander verbindbar sind. Am Vorderbereich können auch vordere Seitenabschnitte vorgesehen sein, welche sich in Querrichtung zumindest über mittlere seitliche Längsränder des Schrittbereichs hinaus erstrecken.

Um bei Anwendung der Inkontinenzwegwerfwindel eine Verbindung in Umfangsrichtung um den Benutzer zwischen Vorderbereich und Rückenbereich zu erhalten, sind die hinteren und/oder vorderen Seitenabschnitten in Querrichtung ausladend ausgestaltet, so dass im ausgebreiteten Zustand einer Inkontinenzwegwerfwindel am hinteren und/oder vorderen Produktende eine durchaus beachtliche Spannbreite vorliegt, insbesondere bei einer Ausführungsform einer Inkontinenzwegwerfwindel als eine Erwachsenen-Inkontinenzwegwerfwindel. Zum Vertrieb oder im Vertrieb dargebotenen Zustand ist eine Inkontinenzwegwerfwindel herstellerseitig zumeist in eine gefaltete Konfiguration gebracht. Dabei sind die Seitenabschnitte beispielsweise auf sich selbst gefaltet und/oder die Seitenabschnitte sind auf die Innenseite der Inkontinenzwegwerfwindel eingefaltet, und optional in einem solchen Zwischenstatus lösbar fixiert, so dass in einer gefalteten Konfiguration der Vorderbereich und der Rückenbereich der Inkontinenzeinwegwerfwindel eine fast annähernd gleiche Quererstreckung aufweisen.

Zum Anlegen einer Inkontinenzwegwerfwindel wird die Inkontinenzwegwerfwindel entlang der Längsrichtung mittig auf die Innenseite zu einem "Schiffchen" gefaltet und in dieser Schiffchen-Konfiguration zwischen den Beinen des Benutzers platziert. Beim Anlegen der Inkontinenzwegwerfwindel an einen stehenden oder einen liegenden Benutzer ist die Anlegetechnik unterschiedlich. Beim stehenden Benutzer werden bereits vor der Schiffchen-Bildung die Seitenabschnitte zumindest teilweise aufgefaltet, hingegen beim liegenden Benutzer wird die Schiffchen-Konfiguration mit den noch darin eingefalteten Seitenabschnitten durch die Beine des Benutzers geschoben und erst dann werden am Rückenbereich die Seitenabschnitte aufgefaltet.

Wegwerfbare absorbierende Inkontinenzwegwerfwindeln weisen entlang der Längsrichtung zumeist unterschiedliche Zonen und Bereiche an Funktionalität auf, so dass die richtige Positionierung der Inkontinenzwegwerfwindel am Benutzer und dabei die Orientierung, welches der beiden Produktenden der Vorderbereich und Rückenbereich sein soll, ganz wesentlich ist.

Einer geschulten Pflegekraft ist es aus ihrer Fachkenntnis und auch Erfahrung bekannt, dass bei derartigen Inkontinenzwegwerfwindel bei Aufsicht auf eine Oberfläche der Inkontinenzwegwerfwindel der größere Oberflächenbereich oftmals dazu bestimmt ist auf die Rückseite des Trägers ausgerichtet zu sein oder womöglich Verschlussmittel ein Hinweis auf ein hinteres Ende der Inkontinenzwegwerfwindel sein können.

Dennoch zeigen die vorgenannten Arbeitsabläufe auf, dass eben kaum ein vollständiges Entfalten und Aufspannen des Produkts zum visuellen Erfassen eines vermeintlich größeren Oberflächenbereichs durchgeführt wird und dies zudem zeitlich aufwändig wäre. Auch aufgrund bei absorbierenden Inkontinenzwegwerfwindeln durchaus optional eingebrachten Elastifizierungsmittel kann eine Dimensions- und damit Flächeneinschätzung aufgrund der mit den Elastifizierungsmittel einhergehenden Raffung erschwert sein.

Auch während des Anlegevorgangs der absorbierenden Inkontinenzwegwerfwindel durch ein mehrmaliges Verändern der Konfiguration der Inkontinenzwegwerfwindel, wie von einer möglicherweisen herstellerseitigen Faltungskonfiguration in eine "Schiffchen"-Konfiguration, dabei eventuell Bedarf eines situationsbedingten Umgreifens mit den Händen (wenn die Pflegeperson den Benutzer der Inkontinenzwegwerfwindel stützen oder umlagern muss) kann eine Irritation in der ursprünglich angedachten Orientierung der Inkontinenzwegwerfwindel auftreten.

Die Problematik zur Unterscheidung zwischen Vorderbereich und Rückenbereich wird noch verschärft, wenn die Anwendung und das Anlegen von solchen Produkten von im Umgang mit Inkontinenzwegwerfwindeln weniger geschulten Personen, oftmals außerhalb von stationären Institutionen, im heimischen privaten Umfeld vorgenommen wird.

Im Stand der Technik ist bekannt, Markierungen für vorne und hinten des jeweiligen Produkts anzubringen. So ist es von regulärer Kleidung und auch regulärer Unterwäsche bekannt, eine Markierung einzubringen, die die Vorderseite oder die Rückseite kennzeichnet. Vor allem findet sich eine derartige Markierung oftmals im hinteren Bereich, so beispielsweise in Form eines eingenähten Etiketts.

Aus der WO 2006/036231 A1 ist bekannt, bei absorbierenden Artikeln mit im Rückenbereich und im Vorderbereich vorhandenen und miteinander verbindbar zu bringenden Verschlusselementen, auf der körperzugewandten Oberseite im Rückenbereich Markierungen vorzusehen, um den Anwender beim Anlegevorgang zu einer richtigen Positionierung der Verschlusselemente anzuleiten, damit die vorderen und hinteren Verschlusselemente im Wesentlichen richtig zueinander ausgerichtet sind.

Die WO 2010/016793A1 zeigt eine Gürtelwindel mit im Rückenbereich auf der Außenseite des absorbierenden Hauptteils aufgebrachten Instruktionen zu den Anlegeverfahrensschritten der Windel.

Das Dokument WO 2009/117492 A2 offenbart einen absorbierenden Artikel, insbesondere eine Windelhose, mit im Bauchbereich und/oder Rückenbereich vorgesehenen mehrlagigen Laminaten, wobei diese Laminate ein ein Kennzeichen aufweisendes nicht-elastomeres Flächenmaterial umfassen, das dabei an eine elastomere Folie gekoppelt zwischen zwei Außenlagen eingebracht ist und wobei dieses Kennzeichen durch beide Außenlagen visuell erkennbar sein kann.

Ausgehend von diesen Erkenntnissen des Standes der Technik, ist es wünschenswert und es ist die Aufgabe der Erfindung, für eine derartige absorbierende Inkontinenzwegwerfwindel eine Markierung vorzusehen, die eine einfache und unaufwändige Differenzierung für den Rückenbereich unterstützt, unabhängig vom Konfigurationszustand der Inkontinenzwegwerfwindel.

Weiter besteht insbesondere auch die Aufgabe eine Markierung für eine Inkontinenzwegwerfwindel bereitzustellen, die eine gute Kennzeichnungskraft aufweist, gleichzeitig jedoch hinreichend dezent ist, wobei zwei Informationen bereitgestellt werden können.

Die Erfindung löst diese Aufgabe durch eine absorbierende Inkontinenzwegwerfwindel mit den Merkmalen des Anspruchs 1:
Dazu ist im Rückenbereich ein Kennzeichnungsmittel vorgesehen, das an der Längsmittelachse angeordnet ist, wobei das Kennzeichnungsmittel in einem ersten Bereich des Rückenbereichs ein erstes Kennzeichen aufweist, das durch eine flächenhaft erstreckte farbige Zone gebildet ist und das eine erste Information bereitstellt, wobei die farbige Zone visuell unterscheidbar ist von einem das erste Kennzeichen umgebenden zweiten Bereich des Rückenbereichs und wobei das Kennzeichnungsmittel zumindest bereichsweise sowohl auf der Innenseite als auch auf der Außenseite visuell erkennbar ist.

Mit der Ausgestaltung des Kennzeichnungsmittels mit einer visuell erkennbaren farbigen Zone, wobei diese zumindest bereichsweise auf der Innenseite und auf der Außenseite visuell erkennbar ist, erhält der Anwender sowohl bei der ersten In-die-Handnahme der Inkontinenzwegwerfwindel als auch über die verschiedenen die Konfiguration der Inkontinenzwegwerfwindel verändernden Zwischenschritte beim Anlegevorgang der Inkontinenzwegwerfwindel einen Hinweis über die Orientierung der Inkontinenzwegwerfwindel.

Durch die Anordnung des Kennzeichnungsmittels an der Längsmittelachse bietet dies dem Anwender einen Hinweis auf die Längsmittelachse der Inkontinenzwegwerfwindel und damit eine Orientierungshilfe für eine mittige Faltung der Inkontinenzwegwerfwindel in Längsrichtung im Wesentlichen um die Längsmittelachse bei der Formung eines "Schiffchen" für den Anlegevorgang.

Vorzugsweise ist das Kennzeichnungsmittel in einem Rückenendabschnitt vorgesehen, insbesondere ist das Kennzeichnungsmittel vollständig in dem Rückenendabschnitt vorgesehen.

Die Anordnung des Kennzeichnungsmittels innerhalb eines hinteren Rückenendabschnitts, der lediglich ein Neuntel (1/9) der Produktlänge L1 erfasst, zeigt sich als vorteilhaft, da für den Anwender die Wahrnehmung des Kennzeichnungsmittels erleichtert ist im Vergleich zu weit in Richtung Quermittelachse positionierten Markierungen.

Mit "Innenseite" ist die dem Körper des Anwenders zugewandte Oberseite der Inkontinenzwegwerfwindel gemeint und "Außenseite" bezeichnet dabei die körperabgewandte Oberseite der Inkontinenzwegwerfwindel.

Unter "Visuell unterscheidbar" wird verstanden, dass die Unterscheidung bereits mit bloßem menschlichen Auge zu erkennen ist, gegebenenfalls kann die Unterscheidbarkeit weiter mit optischen Hilfsmitteln unterstützt werden.

"Visuell wahrnehmbar bzw. visuell erkennbar" ist gleichbedeutend mit "mit bloßem menschlichen Auge erkennbar".

Der verwendete Begriff "Farbe" bzw. "farbig" bezieht sich auf die visuelle Wahrnehmungseigenschaft von aus dem Lichtspektrum abgeleiteten und als solche vom Menschen über das Auge erkennbare Lichtleistung. Farben sind typischerweise bekannt wie u.a. Schwarz, Rot, Blau, Grün, Gelb und Mischungen davon.

Die Farbe kann dabei über den CIEL*a*b*-Farbraum gemessen werden, normiert in der DIN EN ISO 11664-4: 2012-06 Teil 4. Der CIEL*a*b*-Farbraum basiert im Wesentlichen auf der Darstellung einer Farbe innerhalb eines Koordinatensystems mit drei Achsen. Die Achse L* repräsentiert die Helligkeit zwischen 0 (schwarz) - 100 (weiß); die a*-Achse gibt den Grün- oder Rotanteil wider, mit negativen Werten für Grün und positiven Werten für Rot. Die b*-Achse gibt den Blau-oder Gelb-Anteil wider, dabei mit negativen Werten für Blau und positive Werte für Gelb.

Die farbige Zone hebt sich somit bei Aufsicht auf die Innenseite der Inkontinenzwegwerfwindel im Rückenbereich, insbesondere im Rückenendabschnitt und bei Aufsicht auf die Außenseite der Inkontinenzwegwerfwindel im Rückenbereich, insbesondere im Rückenendabschnitt von der visuell vorherrschenden Farbe der jeweiligen Aufsichtsfläche auf der Innenseite und Außenseite im Rückenbereich, insbesondere im Rückenendabschnitt ab.

Die farbige Zone hebt sich insbesondere von der grundlegenden Einfärbung, also einer bereits vor Aufbringung des Kennzeichnungsmittels vorherrschenden Farbe eines im Rückenbereich, insbesondere im Rückenendabschnitt für das Kennzeichnungsmittel vorgesehenen Trägerflachbahnmaterials ab, auf welches eine die farbige Zone bildende Komponente bzw. Material vorgesehen, insbesondere eingebracht und/oder aufgebracht ist.

Die flächenhaft erstreckte farbige Zone kann auf unterschiedliche Weise im Trägerflachbahnmaterial eingebracht sein. So ist es denkbar, dass die farbige Zone durch eine Beschichtung oder einen Aufdruck und/oder auf anderem Wege ausgebildet wird.

Als Trägerflachbahnmaterial für die farbige Zone des Kennzeichnungsmittels sind insbesondere Materiallagen der Inkontinenzwegwerfwindel vorgesehen, die eine die Außenseite und oder Innenseite bildende Lage bilden. Als Trägerflachbahnmaterial sind insbesondere den Saugkörper umgebende Chassismaterialien, insbesondere das Deckblatt-Material und/oder das Backsheet-Material vorgesehen.

Das erste Kennzeichen des Kennzeichnungsmittels ist durch eine flächenhaft erstreckte farbige Zone gebildet. Unter Flächenhaft erstreckt wird dabei verstanden, dass die farbige Zone nicht auf einen einfachen farbigen Punkt oder eine einfache farbige Linie reduziert sein darf. Die flächenhaft erstreckte farbige Zone erscheint dabei als vollflächige farbige Fläche. Dabei kann sich die farbige Zone durchaus als ein erkennbarer Bereich mit einem darin homogen gleichmäßigen Muster an farbigen Bereichen zusammensetzen, wie z.B. einem Punktmuster oder Linienmuster.

Unter "Beschichtung" oder "Aufdruck" wird hierbei jeglicher auf die Oberfläche eines Trägerflachbahnmaterials aufgebrachter Zusatz verstanden, der zu einer visuellen, gegebenenfalls verbunden mit einer haptischen und/oder technischen Veränderung des Trägerflachbahnmaterials führt. Die Beschichtung/der Aufdruck kann als haptisch wahrnehmbar erhoben mit einer gewissen Beschichtungsdicke als einseitig aufliegender Auftrag ausgeführt sein, oder zusätzlich oder ausschließlich als ein das Trägerflachbahnmaterial durchdringender Auftrag vorgesehen sein.

Die im Stand der Technik bekannten Verfahren zum Erhalt einer farbigen Zone umfassen beispielsweise eine Direktbeschichtung mittels Hochdruck, Tiefdruck, Flachdruck, Rotationsdruck oder auch berührungsloses Beschichten wie Besprühen. Farbige Zonen können aber auch durch Einfärbungsprozesse wie Pigmentdruck, Spritzdruck oder Schablonendruck gestaltet werden.

Materialien und oder Komponenten, die einer solchen farbigen Zone zugeordnet sind, können beispielsweise ein Kleber/ Hotmeltauftrag, eine Druckfarbe als Paste oder eine Tinte sein.

Insbesondere bevorzugt ist die farbige Zone des Kennzeichnungsmittels eine Bedruckung, insbesondere eine farbige Bedruckung.

Das Kennzeichnungsmittel, das zumindest bereichsweise sowohl auf der Außenseite als auch auf der Innenseite visuell erkennbar ist, wird vorzugsweise nur durch eine einzige die farbige Zone einbringende Maßnahme bereitgestellt. So ist insbesondere vorgesehen, dass die farbige Zone nur einer einzigen Materiallage zugeordnet ist, also in einer einzigen Materiallage eingebracht, angebracht oder aufgebracht ist.

Eine Längsmittelachse der Inkontinenzwegwerfwindel verläuft in der Längsrichtung und halbiert die maximale Erstreckung der Inkontinenzwegwerfwindel in der Querrichtung. Eine Quermittelachse wird bestimmt, indem die Inkontinenzwegwerfwindel in ihrer maximalen Erstreckung in Längsrichtung halbiert wird. Die Quermittelachse verläuft senkrecht zur Längsmittelachse.

Das Kennzeichnungsmittel ist zumindest "an der Längsmittelachse angeordnet". Damit ist gemeint, dass das Kennzeichnungsmittel visuell erkennbar in nächster Nähe der Längsmittelachse angeordnet ist, was einen Abstand des Kennzeichnungsmittels von der Längsmittelachse bis zu maximal 20% einer maximalen Quererstreckung des Kennzeichnungsmittels noch umfasst. Insbesondere ist das Kennzeichnungsmittel so angeordnet, dass das Kennzeichnungsmittel in seiner maximalen Quererstreckung die Längsmittelachse zumindest tangiert, vorzugsweise überfängt das Kennzeichnungsmittel die Längsmittelachse.

Vorzugsweise ist das Kennzeichnungsmittel hinsichtlich seiner maximalen Quererstreckung E1 im Wesentlichen mittig, insbesondere symmetrisch zur Längsmittelachse angeordnet.

Besonders bevorzugt ist eine im Wesentlichen symmetrisch um die Längsmittelachse angeordnete Positionierung des Kennzeichnungsmittels. Dabei soll im Wesentlichen symmetrisch noch eine Abweichung von der absoluten Symmetrie von maximal 10%, insbesondere maximal 8% und weiter insbesondere maximal 6% umfassen, ermittelt bezüglich einer Verschiebung einer Längsmittellinie des Kennzeichnungsmittels gegenüber der Längsmittelachse der Inkontinenzwegwerfwindel. Hierzu wird der maximale Abstand a zwischen der Längsmittellinie des Kennzeichnungsmittels und der Längsrandseite der Inkontinenzwegwerfwindel, der maximale Abstand b zwischen Längsmittelachse des Inkontinenzartikels und der Längsrandseite der Inkontinenzwegwerfwindel ermittelt und daraus wie folgt berechnet als b-a/b ≤ 10%, insbesondere ≤ 8%, insbesondere ≤ 6%. Abstände a und b werden dabei auf derselben in Querrichtung ausgerichteten Linie bestimmt.

Unter einer maximalen Quererstreckung E1 des Kennzeichnungsmittels versteht sich die Erstreckung des Kennzeichnungsmittels zwischen seinen jeweils äußersten Punkten in Querrichtung der Inkontinenzwegwerfwindel. Unter einer maximalen Längserstreckung F1 des Kennzeichnungsmittels versteht sich die Erstreckung des Kennzeichnungsmittels zwischen seinen jeweils äußersten Punkten in Längsrichtung der Inkontinenzwegwerfwindel.

An dieser Stelle sei ausgeführt, dass in der vorliegenden Anmeldung jegliche Bemessungsangaben hinsichtlich Quererstreckung, Längserstreckung oder Abstand von Abschnitten oder von an sich beliebigen Komponenten des Artikels in eben ausgebreitetem Zustand der den Artikel bildenden Flachmaterialien bestimmt werden. Wenn der Artikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies-Folienverbundbasis oder Folienlage auf natürliche Weise.

Die Produktlänge L1 einer Inkontinenzwegwerfwindel ist die maximale Längserstreckung gemessen in mm in Längsrichtung der Inkontinenzwegwerfwindel zwischen einem hinteren maximal äußersten Abschnitt des hinteren Hüftrandes der Inkontinenzwegwerfwindel bis zu einem vorderen maximalen äußersten Abschnitt des vorderen Hüftrandes der Inkontinenzwegwerfwindel.

Die hintere Produktspannbreite B1 einer Inkontinenzwegwerfwindel ist die maximale Querstreckung gemessen in mm in Querrichtung der Inkontinenzwegwerfwindel im Rückenbereich der Inkontinenzwegwerfwindel, also im Bereich der hinteren Seitenabschnitte.

"Wegwerfbar" bedeutet, dass die absorbierende Inkontinenzwegwerfwindel zum insbesondere einmaligen Gebrauch bestimmt ist. Das heißt eine wegwerfbare absorbierende Inkontinenzwegwerfwindel ist nicht dafür geeignet und vorgesehen nach Gebrauch gereinigt oder gewaschen und anschließend wiederverwendet zu werden.

Der "Überhang" wird verstanden als ein Bereich von Flachbahnmaterialien, die sich außerhalb der Konturlinie des Saugkörpers erstrecken. Insbesondere umfasst der Überhang die Erstreckung des den Saugkörper umgebenden Chassismaterials, insbesondere Deckblatt-Materials und/oder Backsheet-Materials außerhalb der Konturlinie des Saugkörpers oder ist daraus gebildet. Es wird dabei die maximale Erstreckung, also die von der Konturlinie des Absorptionskörpers am weitesten distal gelegene äußere Erstreckung des Chassismaterials, wie Deckblatt-Materials und/oder des Backsheet-Materials herangezogen.

Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln, wie beispielsweise den sogenannten Cuffelementen, sein. Es wird also so verstanden, dass auch bei Verbunden, wie zusammengesetzten Deckblatt-Materialien und/oder Backsheet-Materialien, bei denen die einzelnen Lagen sich nicht kongruent überdecken, bei der Betrachtung des Überhangs die jeweils maximale, also am weitesten distal gelegene äußere Erstreckung des Verbundes bzw. der darin vorkommenden einzelnen Material-Lagen herangezogen wird.

Der Rückenendabschnitt bzw. der Vorderendabschnitt ist ein definierter Bereich der Inkontinenzwegwerfwindel, der ausgehend vom hinteren Hüftrand bzw. vom vorderen Hüftrand in Längsrichtung der Inkontinenzwegwerfwindel jeweils ein Neuntel (1/9) der Produktlänge L1 erfasst. Die Begrenzung des Rückenendabschnitts bzw. des Vorderendabschnitts in lateraler Erstreckung, also in Querrichtung der Inkontinenzwegwerfwindel ist festgelegt durch zwei in Längsrichtung verlaufende Hilfslinien. Jede Hilfslinie wird dazu tangierend an den Punkt des jeweiligen mittleren seitlichen Längsrandes des Schrittbereichs angelegt, in dessen Querrichtung sich die schmalste Stelle im Schrittbereich befindet. Im Falle einer im Schrittbereich nicht vorhandenen schmalsten Stelle, wird die entlang der Quermittelachse vorhandene Distanz zwischen den beiden seitlichen mittleren Längsrändern als Abstand für die beiden Hilfslinien herangezogen. Die Hilfslinien werden fortgeführt bis sie den hinteren Hüftrand bzw. den vorderen Hüftrand kreuzen.

Der hintere Quernahtabschnitt bzw. der vordere Quernahtabschnitt ist der Bereich innerhalb des Rückenendabschnitts bzw. innerhalb des Vorderendabschnitts zwischen einer den Saugkörper umschreibenden Konturlinie und dem jeweiligen hinteren Hüftrand bzw. vorderen Hüftrand.

In einer bevorzugten Ausführungsform der Inkontinenzwegwerfwindel ist vorgesehen, dass der hintere Quernahtabschnitt und/oder der vordere Quernahtabschnitt einen Überhang eines den Saugkörper umgebenden Chassismaterials, insbesondere eines Deckblatt-Materials und/oder eines Backsheet-Materials umfasst oder daraus gebildet ist, wobei der Überhang einen Bereich umfasst oder daraus gebildet ist, in dem das Chassismaterial, insbesondere das Deckblatt-Material und/oder das Backsheet-Material sich außerhalb einer den Saugkörper begrenzenden Konturlinie erstreckt.

Besonders bevorzugt ist dabei, dass im Rückenbereich zwischen einer den Saugkörper begrenzenden Konturlinie und dem hinteren Hüftrand ein hinterer Quernahtabschnitt vorgesehen ist, in dem das Kennzeichnungsmittel zumindest bereichsweise, vorzugsweise vollständig angeordnet ist.

Insbesondere ist vorgesehen, dass das Kennzeichnungsmittel in einem Rückenendabschnittvorgesehen ist, insbesondere innerhalb angeordnet ist.

Die hier in Rede stehende Inkontinenzwegwerfwindel kann unterschiedlich ausgestaltet sein. So können beidseits am Vorderbereich vordere Seitenabschnitte vorgesehen sein, welche sich in Querrichtung zumindest über mittlere seitliche Längsränder des Schrittbereichs hinaus erstrecken.

Es sind aber auch Ausführungen einer Inkontinenzwegwerfwindel denkbar, in denen nur hintere Seitenabschnitte vorgesehen sind.

Die hinteren und/oder vorderen Seitenabschnitte können sich dabei integral aus dem Hauptteil und dessen bildenden Material, insbesondere Chassismaterialien bilden oder solche umfassen oder können als davon separate Komponente an den Hauptteil angefügt sein. Insbesondere weist der Hauptteil im Rückenbereich hintere seitliche Längsränder und im Vorderbereich vordere seitliche Längsränder auf, wobei zumindest an den hinteren seitlichen Längsrändern hintere Seitenabschnitte angefügt sind, die sich in Querrichtung über die hinteren seitlichen Längsränder hinauserstrecken. Im Falle einer bevorzugten Ausführung, dass auch vordere Seitenabschnitte vorgesehen sind, sind die vorderen Seitenabschnitte an den vorderen seitlichen Längsränder angefügt und erstrecken sich in Querrichtung über die vorderen seitlichen Längsränder des Hauptteils hinaus. In einer alternativen bevorzugten Ausführung sind die hinteren Seitenabschnitte an den hinteren seitlichen Längsrändern des Hauptteils angefügt und erstrecken sich über die hinteren seitlichen Längsränder des Hauptteils hinaus erstrecken und wohingegen an den Vorderbereich keine Seitenabschnitte angefügt sind, sondern die vorderen seitlichen Längsränder des Hauptteils jeweils einen frei endenden Längsrand der Inkontinenzwegwerfwindel bilden.

Das Kennzeichnungsmittel weist erfindungsgemäß ein erstes Kennzeichen auf, das durch eine flächenhaft erstreckte Zone gebildet ist.

Das Kennzeichnungsmittel kann insbesondere eine Anordnung von zumindest zwei Sub-Kennzeichnungsmitteln, weiter insbesondere eine Vielzahl an Sub-Kennzeichnungsmittel aufweisen.

Die funktionale Verbundenheit von mehreren Sub-Kennzeichnungsmittel zu einem Kennzeichnungsmittel ist dadurch gegeben, dass die von zwei Sub-Kennzeichnungsmittel benachbarten farbigen Zonen der jeweiligen ersten Kennzeichen auf einer parallel zur Quermittelachse in Querrichtung der Inkontinenzwegwerfwindel verlaufenden Linie angeordnet sind und dass bei einem ersten Sub-Kennzeichnungsmittel mit einer ersten maximalen Quererstreckung E1a und bei einem benachbarten zweiten Sub-Kennzeichnungsmittel mit einer zweiten maximalen Querstreckung E1b, ein Abstand M zwischen den zwei Sub-Kennzeichnungsmittel als die kürzeste Distanz in Querrichtung zwischen den benachbarten farbigen Zonen höchstens 25 %, insbesondere höchstens 20%, insbesondere höchstens 15%, insbesondere höchstens 10% der ersten oder zweiten maximalen Quererstreckung E1a oder E1b beträgt, dabei im Falle von unterscheidbarer ersten und zweiten Querstreckung E1a und E1b auf die kleinere maximale Quererstreckung von beiden maximalen Querstreckung E1a und E1b bezogen ist.

"Auf einer zur Quermittelachse parallelen Linie angeordnet" versteht sich als Positionierung der farbigen Zone, bei der diese Linie die farbige Zone in ihrer flächenhaften Erstreckung zumindest tangiert, vorzugsweise durchkreuzt.

Insbesondere kann ein an der Längsmittelachse angeordnetes Kennzeichnungsmittel eine Anordnung aus zwei Sub-Kennzeichnungsmittel mit je einem ersten Kennzeichen aufweisen. Die zwei Sub-Kennzeichnungsmittel gleichen sich dabei insbesondere hinsichtlich eines Abstandes N1, N2 des einzelnen Sub-Kennzeichnungsmittels zur Längsmittelachse und/oder insbesondere hinsichtlich einer maximale Querstreckung E1a, E1b des jeweiligen Sub-Kennzeichnungsmittels und /oder insbesondere hinsichtlich einer vom jeweiligen Sub-Kennzeichnungsmittel eingeschriebenen Fläche und/oder insbesondere hinsichtlich einer vom ersten Kennzeichens eingeschriebenen Fläche und Kontur, also Form der farbigen Zone 73a, 73 b. Insbesondere sind die zwei Sub-Kennzeichnungsmittel im Wesentlichen zueinander symmetrisch an der Längsmittelachse angeordnet.

Der jeweilige Abstand N1, N2 des einzelnen Sub-Kennzeichnungsmittels zur Längsmittelachse beträgt höchstens 20%, insbesondere höchstens 15%, insbesondere höchstens 10% der maximalen Querstreckung des Sub-Kennzeichnungsmittels.

Insbesondere bevorzugt ist vorgesehen, dass das Kennzeichnungsmittel ein Einzelelement ist. D.h. dass das Kennzeichnungsmittel nur ein einziges erstes Kennzeichen gebildet aus einer flächenhaft erstreckten farbigen Zone aufweist.

Das Kennzeichnungsmittel betrifft bezüglich des ersten Kennzeichens insbesondere zusammenhängende Flächen, und weist als Randumfangslinie insbesondere geschlossene Linien, die somit Flächen ausbilden, bevorzugt, wie beispielsweise Kreise, Ellipsen, Ovale, oder aber auch polygone Flächen, also Vielecke auf. D.h., es handelt sich bevorzugt um eine geometrische Figur, die durch einen geschlossenen Streckenzug umschlossen ist. Geschlossene Streckenzüge unterstützen vorteilhaft die visuelle Wahrnehmbarkeit des Kennzeichnungsmittels.

Das Kennzeichnungsmittel und damit das erste Kennzeichen kann dabei in der Ausbildung seiner Randumfangslinie eine einfache geometrische Form besitzen, insbesondere in Form einer Ellipse, eines Ovals oder eines Kreises oder eines Rechteckes oder eines Polygons ausgebildet sein.

Insbesondere bevorzugt kann die Inkontinenzwegwerfwindel mit unterscheidbaren, unterschiedlichen Formen hinsichtlich des Kennzeichnungsmittels bzw. des ersten Kennzeichens versehen sein. Aus unterschiedlichen Formen kann eine Differenzierung der Inkontinenzwegwerfwindel als Unisex-, Männer und/oder Frauen-Inkontinenzwegwerfwindel erhalten werden. So kann vorzugsweise ein Rechteck für ein Männerprodukt und eine Ellipse oder ein Oval für ein Frauenprodukt eingesetzt werden und eine davon unterscheidbare Form für ein Unisexprodukt.

Die Ausführung des Kennzeichnungsmittels ist dergestalt, dass das Kennzeichnungsmittel ein zweites Kennzeichen umfasst, das eine zweite Information bereitstellt, und wobei das zweite Kennzeichen dadurch gebildet ist, dass in dem ersten Kennzeichen Aussparungen der farbigen Zone vorgesehen sind.

Auf diese Weise kann ein zweites Kennzeichen vorgesehen werden, ohne eine weitere farbige Zone einbringen zu müssen, die die Gefahr birgt, dass die verschiedenen Zonen nicht sauber getrennt werden können. Vielmehr wird das zweite Kennzeichen durch die visuell wahrnehmbare Beschaffenheit des innerhalb des vom Kennzeichnungsmittel überfangenden ersten Bereichs gebildet, wobei das zweite Kennzeichen sich visuell wahrnehmbar von der farbigen Zone abhebt. Hierdurch können vergleichsweise einfach und kostengünstig zwei Informationen durch ein Kennzeichnungsmittel bereitgestellt werden, die keinen zusätzlichen Arbeitsschritt, beispielsweise Einnähen eines Etiketts, benötigen, und dadurch, dass das zweite Kennzeichen durch Aussparungen gebildet wird, die innerhalb der farbigen Zone des ersten Kennzeichens angeordnet sind, können damit in gut erkennbarer und doch dezenter Weise verschiedene Informationen bereitgestellt werden.

Insbesondere durch das Einschließen innerhalb einer farbigen Zone wird die zweite Information des zweiten Kennzeichens besser lesbar, als wenn etwa eine herkömmliche Bedruckung ohne den umschließenden Bereich des ersten Kennzeichens vorgesehen wäre. Durch den sich vom zweiten Bereich des Materials visuell abhebenden ersten Bereich des ersten Kennzeichens wird ein Kontrast geschaffen, der es ermöglicht, eine vergleichsweise klein dimensionierte Ausbildung des zweiten Kennzeichens bezüglich der Wahrnehmbarkeit zu verbessern.

Insbesondere können die Aussparungen inselförmig sein. Unter "inselförmige" Aussparungen sollen dabei insbesondere solche Aussparungen verstanden werden, die von einem hinreichend breiten Rahmen der farbigen Zone umgeben sind, da dies die Erkennbarkeit der zweiten Information verbessert. Ebenfalls ist unter "inselförmigen" Aussparungen auch eine solche zu verstehen, die durch das Zusammenziehen mehrerer für sich stehender alphanummerischer oder ansonsten für den menschlichen Verstand als separat wahrnehmbare Zeichen zu einer Aussparung gebildet wird.

Die Aussparungen, insbesondere "inselförmigen" Aussparungen, weisen insbesondere alphanumerische Zeichen auf, wie u.a. Buchstaben, Ziffern oder Sonderzeichen.

Besonders bevorzugt umfasst das zweite Kennzeichen alphanummerische Zeichen, insbesondere in einer Anordnung der alphanummerischen Zeichen mit einer auf der Innenseite erkennbaren Leserichtung. Alternativ sind die alphanummerischen Zeichen bevorzugt in einer Anordnung mit einer auf der Außenseite erkennbaren Leserichtung.

Mit der Ausgestaltung der Ausnehmungen als alphanumerische Zeichen, dessen informativen Inhalt und Kontext der Anwender durch intuitive Lesen erfasst, wird die Aufmerksamkeit eines Anwenders noch mehr auf das Kennzeichnungsmittel gelenkt und damit die Wahrnehmungskraft des Kennzeichnungsmittels unterstützt.

Insbesondere ist vorgesehen, dass die visuell wahrnehmbare Beschaffenheit des Flachbahnmaterials innerhalb der Aussparungen derjenigen visuell wahrnehmbaren Beschaffenheit des Flachbahnmaterials des zweiten Bereiches, also dem das Kennzeichnungsmittel umgebenden Bereich identisch ist.

Dabei kann insbesondere vorgesehen sein, dass das Kennzeichnungsmittel auf dem Backsheet-Material, dabei auf einer der Innenseite zugewandten Oberseite oder einer der Außenseite zugewandten Oberseite des Backsheet-Materials angebracht ist, oder insbesondere bei einem Backsheet-Material umfassend eine innere Folienlage und eine äußere Vlieslage das Kennzeichnungsmitteldazwischen und dabei insbesondere auf der der Außenseite zugewandten Oberseite der Folienlage angebracht ist oder dass das Kennzeichnungsmittel auf dem Deckblatt-Material, dabei insbesondere auf einer der Außenseite zugewandten Oberseite des Deckblatt-Materials angebracht ist.

Hierdurch wird erreicht, dass kein Abrieb der farbigen Zone erfolgt, als auch Hautfreundlichkeit durch Vermeidung von direktem Hautkontakt beibehalten wird.

Unterstützt wird die visuelle Erkennbarkeit des Kennzeichnungsmittels auf der Außenseite und Innenseite dadurch, dass zwischen dem Kennzeichnungsmittel und der jeweiligen Außenseite und Innenseite in dem ersten Bereich, in dem das Kennzeichnungsmittel angeordnet ist, vorzugsweise nur eine geringe Anzahl an Materiallagen vorgesehen ist, ganz besonders bevorzugt unterscheiden sich die Anzahl der Lagen zwischen dem Kennzeichnungsmittel zur Innenseite und der Anzahl der Lagen zwischen dem Kennzeichnungsmittel zur Außenseite höchstens um 2 Lagen, insbesondere höchstens um 1 Lage.

Der Wahrnehmung des Kennzeichnungsmittels innerhalb des Rückenbereichs, insbesondere des Rückenendabschnitts ist es insbesondere zuträglich, wenn bei Betrachtung der Inkontinenzwegwerfwindel auf der Innenseite oder bei Betrachtung der Inkontinenzwegwerfwindel auf der Außenseite ein Unterschied an Ausgestaltung innerhalb des Rückenendabschnitts und Vorderendabschnitt vorliegt, indem der Vorderendabschnitt entweder frei ist von farbiger Markierung oder im Falle einer farbigen Markierung dann eine für den Anwender unterscheidbare weniger prägnante Ausgestaltung der farbigen Markierungen im Vorderendabschnitts gegeben ist im Vergleich zur farbigen Zone des Kennzeichnungsmittels im Rückenendabschnitt.

Um diese visuelle Erkennbarkeit und das visuelle wahrnehmbare Abheben des Kennzeichnungsmittels des Rückenbereichs, insbesondere des Rückenendabschnitts weiter zu verbessern, werden definiert verortete Positionen an definierten Bereichen, ausgedrückt in Form von imaginären Rechtecken, im Rückenendabschnitt und Vorderendabschnitt auf einer jeweiligen Oberseite, d.h. der Außenseite bzw. der Innenseite verglichen:
Dabei kann bevorzugt vorgesehen sein, dass auf der Innenseite oder dass auf der Außenseite das Kennzeichnungsmittel eine Fläche A1 mit einer maximalen Querstreckung E1 überfängt, und dass das Kennzeichnungsmittel von einem ersten imaginären Rechteck gerade einbeschrieben ist, wobei das erste imaginäre Rechteck erste und zweite Längsseiten parallel zur Längsrichtung und eine erste innere Querseite und eine erste äußere Querseite parallel zur Querrichtung aufweist, wobei die erste innere Querseite einen ersten Abstand D1 vom hinteren Hüftrand aufweist und wobei die erste und zweite Längsseite eine Länge Z1 aufweisen, und dass ein zweites imaginäres Rechteck auf derselben Innenseite oder Außenseite in einem Vorderendabschnitt verortbar ist, wobei das zweite imaginäre Rechteck eine dritte und eine vierte Längsseite parallel zu der Längsrichtung aufweist, und eine zweite innere Querseite und eine zweite äußere Querseite parallel zu der Querrichtung aufweist, und wobei die dritte und vierte Längsseite eine Länge Z1' aufweisen, die der Länge Z1 entspricht (Z1'=Z1) und wobei die zweite innere Querseite einen Abstand D2 von einem vorderen Hüftrand aufweist, und dass für jede der Positionen, in die das zweite imaginäre Rechteck verortet wird, gilt:
a) entweder ist innerhalb des zweiten imaginären Rechtecks keine visuell erkennbare farbige Markierung vorhanden, oder
b) im Falle einer visuell erkennbaren farbigen Markierung innerhalb des zweiten imaginären Rechtecks die farbige Markierung eine Fläche A2 mit einem Flächenanteil von höchstens 25%, insbesondere höchstens 20%, weiter insbesondere höchstens 15%, weitere insbesondere höchstens 10 % der Fläche A1 aufweist und/oder
c) im Falle einer visuell erkennbaren farbigen Markierung innerhalb des zweiten imaginären Rechtecks, die farbige Markierung eine maximale Querstreckung E2 aufweist, wobei die maximale Querstreckung E2 kleiner ist als die maximale Quererstreckung E1 des Kennzeichnungsmittels, wobei insbesondere die maximale Quererstreckung E2 höchstens 40 %, insbesondere höchstens 30% %, weiter insbesondere höchstens 20%, weitere insbesondere höchstens 10 % der maximalen Querstreckung E1 beträgt.

Dabei sind die Positionen, in die das zweite imaginäre Rechteck verortet wird, dadurch begrenzt, dass das zweite imaginäre Rechteck im Vergleich zum ersten imaginären Rechteck parallel zur der Längsmittelachse verschoben ist und für den Abstand D2 gilt:
a) D2 ≤ 1,5 x D1 (Abstand D2 weist einen Wert von maximal das eineinhalb-fache (1,5 x) des Wertes von D1 auf) und
b) dabei die zweite äußere Querseite des zweiten imaginären Rechtecks sich nicht über den vorderen Hüftrand hinauserstreckt.

Sofern der hintere bzw. vordere Hüftrand von einer im Wesentlichen zur Quermittelachse parallelen Erstreckung abweicht, wird hierbei der äußerste Punkt der maximalen Erstreckung der Inkontinenzwegwerfwindel in ihrer Längsrichtung des Hauptteils, d.h. ein äußerster Punkt des Hauptteils im Rückenendabschnitt bzw. Vorderendabschnitt verstanden. Durch diesen äußersten Punkt wird eine zur Quermittelachse parallele Hilfsachse durchgelegt und dann der Abstand D1 bzw. D2 als Distanz in Längsrichtung zwischen der ersten inneren Querkante bzw. zwischen der zweiten inneren Querkante und der jeweiligen nächstgelegenen Hilfsachse bestimmt.

Mit einer Asymmetrie an visuell wahrnehmbarer farbiger Ausgestaltung zwischen einer im Rückenendabschnitt durch das Kennzeichnungsmittel und dessen farbiger Zone eingenommenen Fläche und einem entsprechenden Bereich im Vorderendabschnitt wird der Anwender vorteilhaft in der visuellen Wahrnehmbarkeit des Kennzeichnungsmittels im Rückenendabschnitt unterstützt.

Die Unterschiede in einer maximalen Quererstreckung E1 des Kennzeichnungsmittels im Rückenendabschnitt und einer maximalen Querstreckung E2 einer farbigen Markierung im Vorderendabschnitt sind alternativ oder ergänzend zur Flächenbetrachtung für die visuelle Unterscheidungs- und Wahrnehmungskraft des Kennzeichnungsmittels vorteilhaft. Eine Quererstreckung des Kennzeichnungsmittels bzw. einer farbigen Markierung in Querrichtung und damit senkrecht zur Längsrichtung der Inkontinenzwegwerfwindel wird vom Anwender bei an sich zumeist aufgrund einer die Produktspannbreite B1 übertreffenden größeren Produktlänge L1 und dadurch als eher langgestreckt erscheinenden Inkontinenzwegwerfwindeln als auffallendes Merkmal erkannt als im Vergleich bei derart langgestreckt erscheinenden Inkontinenzwegwerfwindeln eine Längserstreckung des Kennzeichnungsmittel bzw. einer Markierung in Längsrichtung der Inkontinenzwegwerfwindel wahrgenommen wird.

Auch in Anbetracht von im Anwendungsvorgang vorgenommenen Konfigurationsänderungen der Inkontinenzwegwerfwindel, so z.B. bei der Schiffchen-Konfiguration durch Umfalten entlang der Längsrichtung, wodurch eine Langerstreckung des Produkts noch weiter betont wird, trägt ein dazu in Querrichtung ausgerichtetes Kennzeichnungsmittel vorteilhaft zu dessen Wahrnehmung bei.

Insbesondere ist vorgesehen, dass das Kennzeichnungsmittel bzw. die farbige Zone des ersten Kennzeichens des Kennzeichnungsmittels eine maximale Quererstreckung E1 von höchstens 1/2, insbesondere höchstens 1/3, insbesondere höchstens ¼ einer Quererstreckung C an einer in der Querrichtung schmalsten Stelle im Schrittbereich, und insbesondere mindestens 1/6 einer Querstreckung an einer in der Querrichtung schmalsten Stelle im Schrittbereich aufweist.

Mit einer gewissen Limitierung des Kennzeichnungsmittels in seiner maximalen Quererstreckung bei einer der Längsmittelachse zugewandten Anordnung des Kennzeichnungsmittels wird für den Anwender die Wahrnehmbarkeit des Kennzeichnungsmittels als ein Indikator für den hinteren Bereich der Inkontinenzwegwerfwindel vorteilhaft unterstützt, zudem aus herkömmlicher Unterwäsche bekannten Markierung im Rückenbereich ein farbige Zone im Rückenendabschnitt vom Anwender als weniger unangenehm wahrgenommen wird.

Weiter bevorzugt weist das Kennzeichnungsmittel eine maximale Längserstreckung F1 von höchstens 1/9, bevorzugt höchstens 1/10 der Produktlänge L1, und insbesondere mindestens 1/36, insbesondere mindestens 1/30 der Produktlänge L1 auf.

Mit einer Limitierung der maximalen Längserstreckung des Kennzeichnungsmittels wird die Prägnanz des Kennzeichnungsmittels für den hinteren Produktbereich unterstützt, als weiter in Richtung Quermittelachse reichende Markierungen für den Anwender andere Assoziationen oder Hinweise darstellen könnten, die der Anwender beispielsweise in einen funktionellen Zusammenhang mit dem Schrittbereich bringen könnte und somit von der Wahrnehmbarkeit des Kennzeichnungsmittels und dessen Funktion als Indikator für den Rückenbereich ablenken würden.

Bei der Ausgestaltung des Kennzeichnungsmittels ist insbesondere vorgesehen, dass eine maximale Querstreckung E1 des Kennzeichnungsmittels in Querrichtung der Inkontinenzwegwerfwindel größer ist als eine maximale Längserstreckung F1 des Kennzeichnungsmittels in Längsrichtung der Inkontinenzwegwerfwindel.

Bei der Handhabung einer an sich ohnehin länglich erscheinenden Inkontinenzwegwerfwindel, zumal mit nicht vollständig entfalteten Seitenabschnitten wirkt ein Kennzeichnungsmittel mit einer in Querrichtung der Inkontinenzwegwerfwindel ausgerichteten vergleichsweise größeren maximalen Querstreckung als dessen Längserstreckung in Längsrichtung der Inkontinenzwegwerfwindel prägnanter und dem Kennzeichnungsmittel kommt damit eine größere Wahrnehmungskraft zu.

Bevorzugt ist das Kennzeichnungsmittel mit einer maximalen Längserstreckung F1 mit mindestens 50% der maximalen Längserstreckung F1, vorzugsweise mindestens 60%, weitere vorzugsweise mindestens 70%, weiter vorzugsweise mindestens 80%, weiter vorzugsweise mindestens 90% innerhalb eines hinteren Quernahtabschnitts angeordnet, insbesondere bevorzugt ist das Kennzeichnungsmittel vollumfänglich innerhalb eines hinteren Quernahtabschnitts angeordnet.

Diese zumindest bereichsweise Positionierung des Kennzeichnungsmittels innerhalb eines hinteren Quernahtabschnitts, welcher definitionsgemäß keinen Saugkörper aufweist, unterstützt vorteilhaft die visuelle Erkennbarkeit des Kennzeichnungsmittels sowohl auf der Außenseite und der Innenseite der Inkontinenzwegwerfwindel. Eine zunehmende Anordnung des Kennzeichnungsmittels ausschließlich innerhalb des hinteren Quernahtabschnitts und die damit einhergehende Übereinstimmung der visuell wahrnehmbaren Flächenabdeckung des Kennzeichnungsmittels auf der Innenseite und der Außenseite, erleichtert für den Anwender das Erkennen des Kennzeichnungsmittels unabhängig von der in der Anwendung bedingten Konfigurationsänderungen der Inkontinenzwegwerfwindel zwischen Außenseite und Innenseite. Zudem wird durch Positionierung des Kennzeichnungsmittels innerhalb eines Bereiches der Inkontinenzwegwerfwindel mit einem gleichen Lagenaufbau ein einheitliches Erscheinungsbild des Kennzeichnungsmittels geschaffen, was dessen Wahrnehmbarkeit unterstützt.

Es ist insbesondere vorgesehen, dass das Kennzeichnungsmittel außerhalb eines Überlappungsbereiches mit dem Saugkörper vorgesehen ist. Hierdurch wird erreicht, dass das Kennzeichnungsmittel nicht in einem Bereich vorgesehen ist, der aufgrund der Materiallagenhäufung eine relative hohe Opazität aufweist.

Die Inkontinenzwegwerfwindel kann unterschiedliche Formen aufweisen, wobei die Form durch die äußere Kontur, also die Konturlinie der Inkontinenzwegwerfwindel gegeben ist.

Die Inkontinenzwegwerfwindel kann über deren Produktlänge L1 unterschiedliche Eigenschaften und damit auch unterschiedliche Funktionsbereiche aufweisen.

Insbesondere kann eine mittels der Quermittelachse abgegrenzte vordere und hintere Produktlängshälfte zumindest hinsichtlich einer Eigenschaft nicht spiegelsymmetrisch bezogen auf die Quermittelachse sein, dabei kann die Eigenschaft aus der Gruppe enthaltend Fläche des Produkts, Fläche des Saugkörpers, Lagenaufbau des Saugkörpers, Saugleistung des Saugkörpers, chemische Zusammensetzung des Saugkörpers entnommen sein.

Der Saugkörper ist geeignet und dazu bestimmt Körperausscheidungen, insbesondere Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5- 50 Gewichtsprozent, vorzugsweise zu 10 - 40 Gewichtsprozent bezogen auf das Gesamtgewicht des Saugkörpers. Das SAP-Material kann beispielsweise partikelförmig oder faserförmig oder blatt- oder schaumförmig ausgebildet sein. Das SAP-Material kann insbesondere zumindest das 15-fache, insbesondere das 20-fache seines Gewichts an 0,9 gewichtsprozentiger Kochsalzlösung absorbieren (gemessen nach NWSP 242.0.R2(15)). Im Saugkörper können alternativ oder ergänzend weitere Materialien, wie Zellstofffasern (wood pulp) oder Kunststofffasern enthalten sein.

Der Saugkörper kann auch mehrere Lagen aufweisen, die sich in ihrer materiellen und/oder prozentualen Zusammensetzung (SAP, Fasertyp, chemische Faserzusammensetzung) unterscheiden, und die insbesondere zudem eine in Quer- und auch Längsrichtung unterschiedliche Erstreckung aufweisen können.

Dem Saugkörper nicht zugerechnet werden Lagen, die nicht der dauerhaften Flüssigkeitsspeicherung dienen, sondern der Flüssigkeitsverteilung und Weiterleitung, wie dem Fachmann bekannte Flüssigkeitsverteilerauflagen.

Zur Ermittlung der Saugleistung kann die Inkontinenzwegwerfwindel bzw. deren hintere und vordere Produktlängshälfte nach der ISO 11984-1 (1996) vermessen werden.

Bei derart unterschiedlichen Eigenschaften innerhalb der hinteren und vorderen Produktlängshälfte der Inkontinenzwegwerfwindel ist ein Kennzeichnungsmittel im Rückenbereich, insbesondere Rückenendabschnitt eine für den Anwender wesentliche Hilfestellung zur nutzungsgerechten und der jeweiligen Inkontinenzwegwerfwindel zugeschriebenen Verwendung und Orientierung vorne/hinten.

Die Inkontinenzwegwerfwindel ist insbesondere eine Erwachsenen-Inkontinenzwegwerfwindel. Insbesondere bei mit einer Ausführungsform als eine Erwachsenen-Inkontinenzwegwerfwindel einhergehenden größeren Abmessungen, sowohl in Längsrichtung als auch in Querrichtung und in der gesamten flächenhaften Erstreckung, vermag das Kennzeichnungsmittel durch dessen Positionierung innerhalb des Rückenbereichs, insbesondere Rückenendabschnitts vorteilhaft zu einer Differenzierung zwischen hinterem und vorderen Ende des Produkts beitragen.

Die Inkontinenzwegwerfwindel weist insbesondere eine Produktfläche von 220.000 - 750.000 mm², vorzugsweise 230.000 - 740.000 mm², weiter vorzugsweise 240.000 - 730.000 mm² auf.

Die Produktlänge L1 der Inkontinenzwegwerfwindel beträgt insbesondere 500 - 1200 mm, vorzugsweise 600 - 1100 mm, weiter vorzugsweise 600-1000 mm.

Die Inkontinenzwegwerfwindel weist eine hintere Produktspannweite B1 von insbesondere 350 - 1200 mm, vorzugsweise 400 - 1100 mm, weiter vorzugsweise 450 -1050 mm auf. Die hintere Produktspannbreite umfasst die Querstreckung des Hauptteils zusammen mit den Seitenabschnitten.

Ein Überhang von Deckblatt-Material und/oder Backsheet-Material, das sich außerhalb einer den Saugkörper begrenzenden Konturlinie erstreckt, kann einen Flächenanteil von insbesondere 20-62 %, vorzugsweise 25 - 58 %, weiter vorzugsweise 25-55 % bezogen auf die Produktfläche aufweisen. Insbesondere weist ein Überhang eine Fläche von 90.000 - 550.000 mm², vorzugsweise 95.000 - 540.000 mm², weiter vorzugsweise 100.000 - 530.000 mm² auf.

Vorzugsweise weist ein hinterer Quernahtabschnitt einen Flächenanteil von 2 - 11%, bevorzugt 3 -10 %, weiter bevorzugt 3-9 % bezogen auf die Fläche der Inkontinenzwegwerfwindel auf.

Ein vorderer Quernahtabschnitt kann einen Flächenanteil von 3- 12%, bevorzugt 4 - 11 %, weiter bevorzugt 4 - 10 % bezogen auf Produktfläche der Inkontinenzwegwerfwindel aufweisen.

Vorzugsweise weist ein hinterer Quernahtabschnitt einen Flächenanteil von 6 - 14%, bevorzugt 7 - 13%, weiter bevorzugt 7-12% bezogen auf eine Fläche einer Hauptteil - Mittenzone auf, wobei die Hauptteil-Mittenzone begrenzt ist in Längsrichtung durch den hinteren und vorderen Hüftrand und in Querrichtung durch jeweils an der schmalsten Stelle des Schrittbereichs tangierend angelegte Hilfslinien.

Im Falle einer im Schrittbereich nicht vorhandenen schmalsten Stelle, wird die entlang der Quermittelachse vorhandene Distanz zwischen den beiden seitlichen mittleren Längsrändern als Abstand für die beiden Hilfslinien herangezogen. Die Hilfslinien werden fortgeführt bis sie den hinteren Hüftrand bzw. den vorderen Hüftrand kreuzen.

Insbesondere weist ein hinterer Quernahtabschnitt einen Flächenanteil von 60 - 100%, bevorzugt 65-100% weiter bevorzugt 70-100% bezogen auf den Rückenendabschnitt auf.

Vorzugsweise weist ein hinterer Quernahtabschnitt eine Fläche von 8.000 - 35.000 mm², vorzugsweise 10.000 - 32.000 mm², weiter vorzugsweise 12.000 -30.000 mm² auf.

Der vordere Quernahtabschnitt kann in Flächenanteil und/oder der Fläche gemessen in mm² dem hinteren Quernahtabschnitt entsprechen.

Im Falle eines hinteren Quernahtabschnitts weist dieser somit insbesondere eine gewisse flächenhafte Erstreckung zur Positionierung eines Kennzeichnungsmittels auf. Ausgedrückt als eine Breite des Quernahtabschnitts an einem jeglichen Punkt von der Konturlinie des Saugkörpers, und dabei die kürzeste Distanz in der Ebene der Inkontinenzwegwerfwindel von der Saugkörper-Konturlinie zum hinteren Hüftrand, so beträgt die Minimum Breite des hinteren Quernahtabschnitts vorzugsweise mindestens 30 mm, insbesondere mindestens 35 mm, vorzugsweise mindestens 40 mm, vorzugsweise mindestens 40 mm und die maximale Breite des hinteren Quernahtabschnitts beträgt vorzugsweise höchstens 90 mm, weiter vorzugsweise höchstens 80 mm, weiter vorzugsweise höchstens 70 mm.

Vorzugsweise ist vorgesehen, dass das Kennzeichnungsmittel 25-50%, vorzugsweise 27 - 48 %, weiter vorzugsweise 30 - 45 %, weiter vorzugsweise 33-43 % einer Fläche des hinteren Quernahtabschnitts überfängt, also überlappt.

Vorzugsweise überfängt das Kennzeichnungsmittel 25 -40%, insbesondere 30-35%, weiter insbesondere 32-34% einer Fläche des Rückenendabschnitts (im Sinne von überlappt).

Das Kennzeichnungsmittel weist eine vorzugsweise von einer Randumfangslinie des Kennzeichnungsmittels umschriebene Fläche A1 von vorzugsweise 2.000 - 15.000 mm², insbesondere von 2.500 - 13.000 mm², weiter insbesondere von 3.000 - 12.000 mm², weiter insbesondere 3.500 - 11.000 mm², weiter insbesondere 4.000 - 11.000 mm² auf, wobei die Fläche A1 in einem planen ausgebreiteten Zustand der Inkontinenzwegwerfwindel gemessen ist.

Vorzugweise sind in einem hinteren und/oder vorderen Quernahtabschnitt das Deckblatt-Material und Backsheet-Material adhäsiv, thermisch und/oder mittels Ultraschall miteinander verbunden.

Die Inkontinenzwegwerfwindel kann zwischen einem hinteren und vorderen Quernahtabschnitt, beidseits einen sich in Längsrichtung erstreckenden ersten und einen zweiten Längsnahtabschnitt aufweisen, also einen Bereich zwischen der in Längsrichtung verlaufenden Konturlinie des Saugkörpers und den Längsrändern Inkontinenzwegwerfwindel bzw. des Hauptteils.

Insbesondere bevorzugt sind in einem ersten und zweiten Längsnahtabschnitt, der in Längsrichtung je beidseits der in Längsrichtung verlaufenden Konturlinie des Saugkörpers zwischen einem vorderen und einem hinteren Quernahtabschnitt angeordnet ist, keine farbigen visuell erkennbaren Markierungen vorgesehen.

Das Backsheet-Material umfasst insbesondere eine Folie oder ist daraus gebildet. Die Folie weist insbesondere ein Flächengewicht von 7 - 40 g/m², weiter insbesondere von 9 - 30 g/m², weiter insbesondere von 9-25 g/m² auf.

Die Folie des Backsheet-Materials kann vorteilhaft auch mit einer Vliesbeschichtung versehen sein oder mit einer der Außenseite zugewandten Vlieslage laminiert sein, was eine textile Anmutung der körperabgewandten Außenseite der Inkontinenzwegwerfwindel vermitteln kann. Die Vliesbeschichtung bzw. Vlieslage umfasst oder besteht vorzugsweise aus einem Spinnvlies (S), Meltblownvlies (M), SM-Vlies, SMS-Vlies, SMMS-Vlies, Kardenvlies oder Through Air bonded Kardenvlies, insbesondere einem Spinnvlies aus Polypropylen. Insbesondere weisen die Vlieslage/Vliesbeschichtung des Backsheet-Materials ein Flächengewicht von 5-25 g/m², 7 - 20 g/m², insbesondere von 8 - 15 g/m² auf.

Auf einer der Außenseite zugewandten Oberseite des Backsheet-Materials sind insbesondere keine adhäsiven Haftungsmittel, wie Klebeabschnitte, Klebestreifen vorgesehen.

Das Deckblatt-Material umfasst vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese.

Das Deckblatt-Material kann dabei vorzugsweise nur aus Topsheet-Material gebildet sein.

Das Topsheet-Material umfasst vorzugsweise Vliesstoffmaterialien, wie Spinnvliese, Kardenvliese oder Through Air bonded Kardenvliese. Besonders bevorzugt umfasst das Topsheet-Material Spinnvlies. Die für das Topsheet eingesetzten Vliesstoffmaterialien haben weiter vorteilhafterweise ein Flächengewicht von 5 - 20 g/m², 8 - 20 g/m², weiter vorzugsweise von 10 - 18 g/m², insbesondere vorzugsweise von 12 - 16 g/m². Besonders bevorzugt umfasst das Topsheet ein hydrophilisiertes Spinnvlies, insbesondere aus Polypropylen, insbesondere mit einem Flächengewicht von 12 - 16 g/m².

Weiter vorzugsweise kann das Deckblatt-Material ein Verbund aus Topsheet-Material und Barrieremittel-Material sein. In einer weiteren vorteilhaften Ausbildung ist das Deckblatt-Material ein Verbund aus einem flüssigkeitsdurchlässigen Topsheet-Material mit Längsrändern und angrenzenden Längsrandbereichen und beidseits an den Längsrändern bzw. Längsrandbereichen des Topsheet-Materials in Fügestellen angefügten hydrophoben Barrieremitteln. Durch diesen Verbund werden in einer Inkontinenzwegwerfwindel die bereichsweise unterschiedlichen Anforderungsprofile, nämlich im mittigen Bereich eine Flüssigkeitsaufnahme und an den Randbereichen eine Retardierung des seitlichen Flüssigkeitsaustritts, bereitgestellt.

Das Barrieremittel-Material umfasst vorzugsweise Vliesstoffmaterialien, wie Spinnvliese, Meltblownvliese, insbesondere bevorzugt Laminate aus einer oder mehreren Spinnvlies(S) und/oder Meltblown(M)-Vlieslagen, insbesondere SMS-Laminate oder SMMS-Laminate, insbesondere auf Basis von Polyolefinen, wie beispielsweise Polyethylen oder Polypropylen.

Die für das Barrieremittel eingesetzten Vliesstoffmaterialien haben weiter vorteilhafterweise ein Flächengewicht von 5 - 20 g/m², vorzugsweise von 8 - 18 g/m² , weiter vorzugsweise von 10 - 15 g/m².

Weiter vorzugsweise kann die Inkontinenzwegwerfwindel auf der Innenseite beidseits jeweils ein eine seitliche Auslaufsperre bildende und/oder beidseits entlang einer Längserstreckung des Saugkörpers verlaufendes Cuffelement aufweisen. Das Cuffelement umfasst vorzugsweise ein Vliesmaterial, insbesondere ein hydrophobes Vliesmaterial, oder ist daraus gebildet. Das Flächengewicht des hierfür eingesetzten Vliesmaterials beträgt vorzugsweise 5 - 20 g/m², vorzugsweise von 8 - 18 g/m² , weiter vorzugsweise von 10 - 15 g/m².

Das Cuffelement -Material kann das vorgenannte Barrieremittel-Material umfassen oder daraus gebildet sein.

Weiter vorzugsweise weisen die Cuffelemente eine Cuffelastifizierung auf, um ein sicheres Emporerheben und Aufstellen der Cuffelemente zu gewährleisten. Eine Elastifizierung erfolgt dabei entweder über eine flächenhafte Elastifizierung der Cuffelemente, indem die Cuffelemente in Längsrichtung elastisch dehnbare Flächenmaterialien umfassen. Insbesondere kann vorgesehen sein, dass in die Cuffelemente im Bereich ihres freien emporerhebbaren Längsrandes im Wesentlichen fadenförmige Elastifizierungsmittel eingesetzt werden oder streifenförmige Elastifizierungsmittel.

Vorzugsweise erstrecken sich Deckblatt-Material und/oder Backsheet-Material vorzugsweise zumindest in Längsrichtung der Inkontinenzwegwerfwindel, weitere vorzugsweise in Querrichtung der Inkontinenzwegwerfwindel über Konturränder /die Konturlinie des Saugkörpers hinaus, um einen entsprechenden Überhang zu bilden. In dem Überhang sind Backsheet-Material und Deckblatt-Material vorzugsweise zumindest bereichsweise miteinander verbunden, insbesondere durch an sich bekannte Fügeverfahren wie Schweißen, Siegeln, Nähen oder Kleben.

Vorzugsweise weist die Inkontinenzwegwerfwindel, insbesondere im Schrittbereich, in Längsrichtung verlaufende Schrittelastifizierungsmittel auf. Die Schrittelastifizierungsmittel sind insbesondere zumindest abschnittsweise außerhalb der Konturlinie des Saugkörpers angeordnet.

Die Schrittelastifizierungsmittel können elastische Fäden oder auch flachmaterial- oder bandförmige Materialien wie elastische Bänder, Folien, Vliesstoffe oder Schaumstoffe aufweisen. "In Längsrichtung der Inkontinenzwegwerfwindel" bedeutet hierbei, dass das Schrittelastifizierungsmittel zumindest eine Komponente in Längsrichtung der Inkontinenzwegwerfwindel aufweist, mithin auch schräg oder gekrümmt zur Längsrichtung der Inkontinenzwegwerfwindel verlaufen kann. Die mit den Schrittelastifizierungsmittel versehenen Bereiche der Inkontinenzwegwerfwindel können als zusätzliche seitliche Auslaufsperre dienen und gleichzeitig das Anlegen der Inkontinenzwegwerfwindel, insbesondere des mittleren Produktabschnitts gegen den Körper eines Trägers fördern und eine bootförmige Krümmung der Inkontinenzwegwerfwindel hervorrufen.

Vorzugsweise sind im Rückenendabschnitt und/oder Vorderendabschnitt, weiter insbesondere im hinteren und/oder vorderen Quernahtabschnitt keine in die Querrichtung verlaufende Elastifizierungsmittel vorgesehen. Insbesondere sind im Bereich des Kennzeichnungsmittels keine in Querrichtung verlaufende Elastifzierungsmittel vorgesehen. Somit wird der Bereich mit dem Kennzeichnungsmittel keinen durch die Elastifizierung bedingten unterschiedlichen Dehnungszuständen unterworfen und kann somit einen einheitlichen Farbeindruck vermitteln.

Nach einer weiteren bevorzugten Ausführungsform kann vorgesehen sein, dass die Inkontinenzwegwerfwindel zum Vertrieb oder im Vertrieb dargebotenen Zustand herstellerseitig in eine gefaltete Konfiguration gebracht ist. Vorzugsweise ist die gefaltete Konfiguration derart, dass die Innenseite der Inkontinenzwegwerfwindel in der gefalteten Konfiguration keine äußere Oberseite der gefalteten Konfiguration bildet.

In einer bevorzugten gefalteten Konfiguration der Inkontinenzwegwerfwindel sind insbesondere die hinteren Seitenabschnitte und die optional vorhandenen vorderen Seitenabschnitte herstellerseitig wenigstens um eine in Längsrichtung verlaufende Seitenabschnitt-Faltachse auf die Innenseite und/oder auf sich selbst gefaltet, weiter vorzugsweise um zwei, weiter vorzugsweise um drei Seitenabschnitt-Faltachsen.

Auf sich gefaltete Seitenabschnitte können dabei unterschiedlichen Faltungskonfigurationen zugeführt sein, so wie insbesondere im Dokument DE 102004021353 A1 (in Figur 5c dargestellt) oder im Dokument DE 102009022529 A1 (in Figur 5b dargestellt) beschrieben ist. Eine Faltung der Seitenabschnitte kann beispielsweise auch gemäß DE102009036796 A1 oder DE102005035544 A1 durchgeführt sein.

Weiter bevorzugt kann der Hauptteil herstellerseitig mitsamt den auf sich gefalteten hinteren und/oder vorderen Seitenabschnitten um eine erste und eine zweite jeweils in der Längsrichtung verlaufende Hauptteil-Faltachse nach innen auf sich selbst gefaltet sein, derart dass die beidseitigen hinteren Seitenabschnitte in Dickenrichtung in wenigstens teilweiser Überlappung zueinander zu liegen kommen.

Alternativ oder zusätzlich zu einer Einfaltung der Seitenabschnitte in Längsrichtung kann insbesondere vorgesehen sein, dass die Inkontinenzwegwerfwindel herstellerseitig in eine gefaltete Konfiguration verbracht ist, bei der die Inkontinenzwegwerfwindel zumindest um eine in Querrichtung der Inkontinenzwegwerfwindel verlaufende Faltachse gefaltet ist, und die Innenseite der Inkontinenzwegwerfwindel auf sich gefaltet in der gefalteten Konfiguration innen angeordnet ist.

Dabei wird die Inkontinenzwegwerfwindel insbesondere um eine im Wesentlichen entlang der Quermittelachse verlaufenden Faltachse gefaltet.

Weiter kann vorgesehen sein, dass die Inkontinenzwegwerfwindel herstellerseitig in eine gefaltete Konfiguration verbracht ist, bei der die Inkontinenzwegwerfwindel um zwei in Querrichtung verlaufende Faltachsen unter Erhalt eines in Längsrichtung hinteren Faltungsabschnitts, der den das Kennzeichnungsmittel aufweisenden Rückenbereich erfasst, insbesondere den das Kennzeichnungsmittel aufweisenden Rückenendabschnitt umfasst, eines mittleren und eines vorderen Faltungsabschnitts gefaltet ist, wobei der vordere Faltungsabschnitt in der gefalteten Konfiguration zwischen dem hinteren Faltungsabschnitt und dem mittleren Faltungsabschnitt angeordnet ist oder wobei der hintere Faltungsabschnitt in der gefalteten Konfiguration zwischen dem vorderen Faltungsabschnitt und dem mittleren Faltungsabschnitt angeordnet ist.

Über die Gestaltung des Kennzeichnungsmittels kann neben der ersten und/oder der zweiten Information eine weitere Information dadurch transportiert werden, dass die farbige Zone neben der Information, wo der hintere Bereich der Inkontinenzwegwerfwindel sich befindet, eine weitere Information beinhalten kann, indem beispielsweise über die gezielte Auswahl einer Farbe der farbigen Zone des ersten Kennzeichens ein Hinweis auf eine Saugstärke beinhaltet sein kann.

Das erste Kennzeichen kann weitere Information beinhalten, indem beispielsweise über die gezielte Auswahl einer Farbe der farbigen Zone ein Hinweis über eine Verwendung in geschlechtsspezifischer Weise beinhaltet sein kann. So kann beispielsweise ein Kennzeichnungsmittel in einer blauen Farbe auf einen bevorzugten Einsatzbereich für Männer und ein Kennzeichnungsmittel in einer rosa Farbe auf einen bevorzugten Einsatz für Frauen hinweisen oder mit einer weiteren davon verschiedenen Farbe auf die Verwendung als Unisex-Inkontinenzwegwerfwindel hinweisen.

Weiter kann über die Form des Kennzeichnungsmittels bzw. über die Form des ersten Kennzeichens, wie beispielsweise Ellipse versus Rechteck, eine weitere Information vermittelt werden.

Als zweites Kennzeichen können dann durch die Aussparungen weitere Informationen transportiert werden. Durch die Aussparungen in der farbigen Zone des ersten Kennzeichens greift das zweite Kennzeichen die visuell wahrnehmbare Beschaffenheit, wie eben die Farbe des Trägerflachbahnmaterials des zweiten Bereichs auf. Hierdurch wird eine unerwünschte durch visuelle Erscheinung oder Auffälligkeit erzeugte Aufmerksamkeit reduziert, da sich mit einer identischen visuell wahrnehmbaren Beschaffenheit des Trägerflachbahnmaterials in den Aussparungen in Zusammenschau mit dem Trägerflachbahnmaterial des das Kennzeichnungsmittel umgebenden zweiten Bereichs ein gewisses homogenes Erscheinungsbild ergibt.

Dabei ist besonders bevorzugt vorgesehen, dass die farbige Zone ausgehend von einer Randumfangslinie des Kennzeichnungsmittels das zweite Kennzeichen derart umschließt, dass die Größe einer Fläche A3, welche von der Randumfangslinie des Kennzeichnungsmittels und einer davon innerhalb und in einem konstanten ersten Abstand AB1 verlaufenden imaginären inneren Abstandslinie begrenzt wird, mindestens 10%, insbesondere mindestens 15%, insbesondere mindestens 20%, insbesondere mindestens 25% einer Größe der Fläche A1 beträgt, wobei als die Fläche A1 die durch die Randumfangslinie des Kennzeichnungsmittels eingeschriebene Fläche definiert ist. Vorzugsweise beträgt die Fläche A3 höchstens 50%, weiter insbesondere höchstens 45%, weiter insbesondere höchstens 40%, weiter insbesondere höchstens 35%, weiter insbesondere höchstens 30% einer Größe der Fläche A1.

Weiter vorzugsweise beträgt der Flächenanteil des zweiten Kennzeichens an der Fläche A3 höchstens 10%, insbesondere höchstens 7%, weiter insbesondere höchstens 5%, insbesondere höchstens 2% beträgt. Die Bestimmung soll hierbei im vollständig ausgebreiteten Zustand der Inkontinenzwegwerfwindel erfolgen. Die Größen der Flächen werde jeweils in mm² vermessen.

Hierdurch wird eine gute visuelle Wahrnehmbarkeit des ersten als auch des zweiten Kennzeichens ermöglicht.

Durch eine derartige Gestaltung einer Fläche A3, die innerhalb der Fläche A1 liegt, wird der visuelle Eindruck einer zumindest dickeren/breiteren Umrandungsfläche erreicht, die sich in der Art eines Randes um das zweite Kennzeichen erstreckt, wodurch die visuelle Sichtbarkeit und Erkennbarkeit verbessert wird, insbesondere auch bei ansonsten nur schwerlesbaren alphanumerischen Zeichen als zweites Kennzeichen.

Insbesondere kann vorgesehen sein, dass zwischen der Randumfangslinie des Kennzeichnungsmittels, also einer Randumfangslinie der visuell wahrnehmbaren farbigen Zone des ersten Kennzeichens, und einer in einem konstanten ersten Abstand AB1 verlaufenden imaginären inneren Abstandslinie, die einen Abstand von mindestens 2 mm, weiter insbesondere 3 mm, weiter insbesondere mindestens 4 mm und weiter insbesondere mindestens 5 mm aufweist, zumindest abschnittsweise, insbesondere vollumfänglich keine Aussparungen vorgesehen sind.

Um diese visuelle Erkennbarkeit und das visuell wahrnehmbare Abheben des Kennzeichnungsmittels bzw. der farbigen Zone des ersten Kennzeichens von den zweiten Bereichen des Trägerflachbahnmaterials, insbesondere des Deckblatt-Materials und/oder des Backsheet-Materials und als solche das Kennzeichnungsmittel umgebend weiter zu verbessern, kann vorgesehen sein, dass die Größe einer Fläche A4, welche von einer Randumfangslinie des Kennzeichnungsmittels und einer davon außerhalb und in einem konstanten zweiten Abstand AB2 verlaufenden imaginären Abstandslinie begrenzt wird, mindestens 30%,insbesondere mindestens 40%, insbesondere mindestens 50%, insbesondere mindestens 60%, weiter insbesondere mindestens 70% der Größe einer Fläche A1 beträgt, wobei als die Fläche A1 die durch die Randumfangslinie des Kennzeichnungsmittels eingeschriebene Fläche definiert ist, wobei vorzugsweise mindestens 90%, insbesondere mindestens 93%, insbesondere mindestens 95% der Fläche A4 frei von weiteren farbigen Zonen, insbesondere als Beschichtungen und/oder Bedruckungen und/oder Änderungen der dem Trägerflachbahnmaterial, insbesondere dem Deckblatt-Material und/oder Backsheet-Material zu eigenen optischen visuellen und/oder haptischen Beschaffenheit sind.

Die Größen der Flächen werden jeweils in mm² vermessen. Die Bestimmung soll hierbei im vollständig plan ausgebreiteten Zustand der Inkontinenzwegwerfwindel erfolgen.

Darüber hinaus kann insbesondere vorgesehen sein, dass zwischen der Randumfangslinie des Kennzeichnungsmittels, also einer Randumfangslinie der farbigen Zone des ersten Kennzeichens, und einer in einem konstanten zweiten Abstand AB2 verlaufenden imaginären äußeren Abstandslinie, die einen Abstand von mindestens 2 mm, weiter insbesondere mindestens 3 mm, weiter insbesondere mindestens 4 mm und weiter insbesondere mindestens 5 mm, weiter insbesondere mindestens 8 mm, weitere insbesondere mindestens 10 mm aufweist, eine innerhalb dieses zweiten Abstands A2 begrenzten Fläche zumindest abschnittsweise, insbesondere vollumfänglich keine farbige Zone, wie z.B. in Form einer Bedruckung vorgesehen ist.

Weiter kann insbesondere vorgesehen sein, dass das Kennzeichnungsmittel eine durch eine Randumfangslinie eingeschriebene Fläche A1 überfängt, und das zweite Kennzeichen einen Flächenanteil von höchstens 50%, insbesondere höchstens 40%, weiter insbesondere höchstens 30%, weiter insbesondere höchstens 20%, und insbesondere wenigstens 3 %, insbesondere wenigstens 5 %, insbesondere wenigstens 8 % der vom Kennzeichnungsmittel überfangenen Fläche A1 einnimmt. Hierdurch bleibt die visuelle Sichtbarkeit des ersten Kennzeichens bestehen.

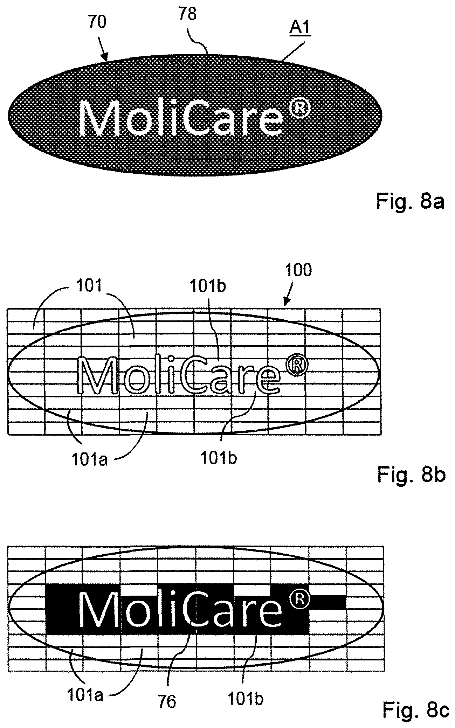

Für die Erkennbarkeit einerseits aber auch die Diskretheit andererseits ist insbesondere eine weitgehend ausgewogene Verteilung des zweiten Kennzeichens innerhalb des ersten Kennzeichens von Vorteil. Die Bestimmung des Verteilungsgrad ist dabei beispielhaft anhand der Figuren 8a-8c erläutert.

Es kann dabei bevorzugt vorgesehen sein, dass wenn das Kennzeichnungsmittel mit einer Randumfangslinie in seinen jeweils äußersten Punkten von einem imaginären Rechteck tangierend eingeschrieben ist, wobei das Rechteck bezüglich seiner Kanten in Längs- und Querrichtung des Inkontinenzartikels angeordnet ist und dabei sowohl in Längsrichtung als auch in Querrichtung in jeweils 10 gleich große Unterrechtecke unterteilt ist, von einer Anzahl N1 an Unterrechtecken, welche vollständig oder zumindest bereichsweise innerhalb der Randumfangslinie lokalisiert sind und davon einer Anzahl N2 derjenigen Unterrechtecken, die zumindest bereichsweise sowohl das erste Kennzeichen als auch das zweite Kennzeichen aufweisen, ein Verteilungsgrad von N2/N1*100% von mindestens 10 %, insbesondere mindestens 20 %, insbesondere mindestens 25%, insbesondere mindestens 30%, insbesondere mindestens 35% vorgesehen ist. Weiter vorzugsweise ist ein Verteilungsgrad von insbesondere höchstens 60%, weiter insbesondere höchstens 55 %, weiter insbesondere höchstens 50%, weiter insbesondere höchstens 45% vorgesehen.

Es ist insbesondere vorgesehen, dass das erste Kennzeichen im ersten Bereich eine erste Farbe aufweist und ein an das erste Kennzeichen direkt angrenzende zweite Bereich eine davon unterscheidbare dritte Farbe aufweist. Weiter insbesondere ist vorgesehen, dass bei einem ein zweites Kennzeichen umfassenden Kennzeichnungsmittel, das erste Kennzeichen eine erste Farbe aufweist und das zweite Kennzeichen eine davon unterscheidbare zweite Farbe aufweist. Insbesondere ist vorgesehen, dass die zweite Farbe und die dritte Farbe annähernd gleich sind.

Insbesondere werden die Farben nach CIEL*a*b* vermessen. Die Messung und Berechnung ist nachfolgend beschrieben.

Insbesondere ist vorgesehen, dass das erste Kennzeichen im ersten Bereich eine erste Farbe aufweist und ein an das erste Kennzeichen direkt angrenzende zweite Bereich eine davon unterscheidbare dritte Farbe aufweist, wobei die Farbe, jeweils auf der Innenseite und/oder jeweils auf der Außenseite nach CIEL*a*b* vermessen ist, und ein nach CIEL*a*b* berechneter Farbabstand zwischen der ersten und der dritten Farbe, dargestellt als Delta E (ΔE*), besteht.

Insbesondere beträgt der nach CIEL*a*b* berechnete Farbabstand zwischen der ersten und der dritten Farbe dargestellt als Delta E (ΔE*) 10 bis 55, vorzugsweise 15 bis 55, weiter vorzugsweise 20 bis 55, weiter vorzugsweise 35 bis 55.

Auf diese Weise wird ein Kennzeichnungsmittel bereitgestellt, dessen farbige Zone des ersten Kennzeichens von dem dieses erste Kennzeichen umgebenden Bereichs gut visuell unterscheidbar ist und dem Kennzeichnungsmittel somit eine gute Wahrnehmbarkeit und damit eine Signalwirkung zukommen kann.

In einer bevorzugten Ausführungsform des Kennzeichnungsmittels, das ein zweites Kennzeichen in Form von Aussparungen innerhalb des ersten Kennzeichens aufweist, ist es insbesondere vorgesehen, dass das erste Kennzeichen im ersten Bereich eine erste Farbe aufweist und das zweite Kennzeichen eine davon unterscheidbare zweite Farbe aufweist, wobei die Farbe, jeweils auf der Innenseite und/oder jeweils auf der Außenseite nach CIEL*a*b* vermessen ist, ein nach CIEL*a*b* berechneter Farbabstand zwischen der ersten und der zweiten Farbe, dargestellt als Delta E (ΔE*), besteht.

Insbesondere beträgt der nach CIEL*a*b* berechnete Farbabstand zwischen der ersten und der zweiten Farbe dargestellt als Delta E (ΔE*) 10 bis 55, vorzugsweise 15 bis 55, weiter vorzugsweise 20 bis 55, weiter vorzugsweise 35 bis 55.

Durch ein sich vom zweiten Kennzeichen abhebendes erste Kennzeichen, der farbigen Zone, wird ein Kontrast geschaffen, der es ermöglicht, die Wahrnehmbarkeit und/oder Lesbarkeit des zweiten Kennzeichens, sogar in einer vergleichsweise klein dimensionierten Ausbildung, zu verbessern.

Insbesondere ist der Farbabstand zwischen erster und zweiter Farbe und der Farbabstand zwischen erster und dritter Farbe im Wesentlichen gleich.

Da das zweite Kennzeichen, in Form von Aussparungen innerhalb der farbigen Zone gebildet ist, und vorzugsweise ohne weitere Oberflächenbehandlung von der Beschaffenheit und/oder Farbe des Materials des dieses erste Kennzeichen umgebenden Bereichs, nämlich zweiten Bereichs gebildet wird, bestimmt dessen Beschaffenheit/Farbe auch den Wert des zweiten Kennzeichens.

Insbesondere auf der Außenseite beträgt ein nach CIEL*a*b* berechneter Farbabstand zwischen der ersten und der zweiten Farbe und/oder zwischen der ersten und dritten Farbe, als Delta E (ΔE*) insbesondere 15 bis 55, vorzugsweise 20 bis 55, weiter vorzugsweise 35 bis 55.

Insbesondere kann ein nach CIEL*a*b* berechneter Farbabstand auf der Außenseite zwischen der ersten und der zweiten Farbe und/oder zwischen der ersten und dritten Farbe größer sein als der auf der Innenseite nach CIEL*a*b* berechnete Farbabstand zwischen der jeweiligen ersten und der zweiten Farbe und/oder der jeweiligen ersten und dritten Farbe.

Dabei unterscheiden sich die jeweiligen Farbabstände von Außenseite und Innenseite, berechnet als Differenz zwischen dem jeweiligen Farbabstand der Außenseite und Innenseite, um einen Betrag von höchstens 20, weiter insbesondere höchstens 18, weiter insbesondere höchstens 16, weiter insbesondere höchstens 14, weiter insbesondere höchstens 12, weiter insbesondere höchstens 10.

Insbesondere kann ein auf der Innenseite nach CIEL*a*b* berechneter Farbabstand zwischen der ersten und der zweiten Farbe und/oder zwischen der ersten und dritten Farbe 15- 40, vorzugsweise 20 - 40 betragen.

Auch in dieser Ausführung ist ein Kontrast innerhalb des Kennzeichnungsmittels zwischen erstem Kennzeichen und dem darin umschlossenen zweitem Kennzeichen und/oder ein Kontrast zwischen der dem Kennzeichnungsmittel zugehörigen farbigen Zone des ersten Kennzeichens und dem das Kennzeichnungsmittel umgebenden Bereichs durchaus vorhanden, um auch auf der Produktinnenseite ein Kennzeichnungsmittel mit Wahrnehmungskraft bereitzustellen.

Der auf der Außenseite zwischen dem ersten und zweiten Kennzeichen im Vergleich zur Innenseite vorzugsweise größere Farbabstand deutet auf einen höheren Kontrast auf der Produktaußenseite hin, was vorteilhaft für die auf der Produktaußenseite durchaus gewünscht höhere Attraktion des Kennzeichnungsmittels ist. Zumal auf der Außenseite oftmals auch andere Markierungen vorhanden sein können, die der Wahrnehmung des Kennzeichnungsmittels in Konkurrenz treten können. Hingegen braucht es auf der Produktinnenseite zumeist keine übersteigerte Attraktion, bei einer auf der Innenseite von einer flächigen Erstreckung des Saugkörpers dominierten Aufsicht, die dabei zumeist einfarbig einheitlich ausgestaltet ist. Dennoch ist das Kennzeichnungsmittel visuell wahrnehmbar und gegenüber der sonstigen Fläche visuell erkennbar.

Vorzugsweise ist vorgesehen, dass in dem ersten Bereich, in dem das Kennzeichnungsmittel und damit die das erste Kennzeichen bildende farbige Zone angebracht ist, zwischen dem Kennzeichnungsmittel und der jeweiligen Außenseite und Innenseite vorzugsweise nur eine geringe Anzahl an Materiallagen vorgesehen ist, ganz besonders bevorzugt unterscheidet sich die Anzahl der Lagen zwischen dem Kennzeichnungsmittel zur Innenseite und der Anzahl der Lagen zwischen dem Kennzeichnungsmittel zur Außenseite höchstens um 2 Lagen, insbesondere höchstens um 1 Lage. Eine entsprechende Verteilung der Lagen ist vorteilhaft auch in dem diesen ersten Bereich umgebenden zweiten Bereich vorgesehen. Dies unterstützt die visuelle Erkennbarkeit des Kennzeichnungsmittels auf der Außenseite und Innenseite.

Vorzugsweise ist vorgesehen, dass die innerhalb des ersten Bereiches des ersten Kennzeichens vorhandenen Materiallagen eine jeweilige Opazität nicht übersteigen, um eine visuelle Wahrnehmung des Kennzeichnungsmittels sowohl auf der Innenseite als auch auf der Außenseite zu ermöglichen.

Die Opazität ist ein Maß für die Lichtundurchlässigkeit einer Materiallage.

Bevorzugt weisen im Bereich des Kennzeichnungsmittels vorgesehene Materiallagen eine Opazität von höchstens 60%, weiter vorzugsweise höchstens 50%, weiter vorzugsweise höchstens 40%, weiter vorzugsweise höchstens 30% auf. Insbesondere bevorzugt weist das Deckblatt-Material eine Opazität von höchstens 60%, weiter vorzugsweise höchstens 50%, weiter vorzugsweise höchstens 40%, weiter vorzugsweise höchstens 30% auf. Weiter vorzugweise weist das Backsheet-Material eine Opazität von höchstens 70%, weiter vorzugsweise höchstens 60%, weiter vorzugsweise höchstens 50%, weiter vorzugsweise von 40%, weiter vorzugsweise von 30% auf.

Dabei erfolgt die Farbmessung nach CIEL*a*b* wie folgt:
**Farbmessung nach dem CIEL*a*b*-Farbraum und daraus abgeleitete Farbabstände, sogenannte ΔE*-Werte:**

Die Farbmessung nach L*a*b*, auch genannt CIEL*a*b*-Farbraum, beschreibt und definiert Farben geräteunabhängig. Der CIEL*a*b*-Farbraum ist genormt in der DIN EN ISO 11664-4: 2012-06 Teil 4.

Der CIEL*a*b*-Farbraum basiert im Wesentlichen auf der Darstellung einer Farbe innerhalb eines Koordinatensystems mit drei Achsen. Die Achse L* repräsentiert die Helligkeit zwischen 0 (schwarz) - 100 (weiß); die a*-Achse gibt den Grün- oder Rotanteil wider, mit negativen Werten für Grün und positiven Werten für Rot. Die b*-Achse gibt den Blau-oder Gelb-Anteil wider, dabei mit negativen Werten für Blau und positive Werte für Gelb.

### Prüfgerät

Für die Farbmessungen wird als Prüfgerät ein Spektralphotometer eingesetzt, wie z.B. das Spectro-guide sphere gloss von BYK-Gardner.

### Probenvorbereitung:

Die Messprobe bzw. den Artikel auf einer ebenen Fläche flach ausbreiten. Für die Probengröße ist letztlich die von der Messfeldbegrenzung des Prüfgerätes vorgegebene Messfläche zu beachten.

Die Messungen wird im plan ausgebreiteten Zustand der Messprobe bzw. des Artikels vorgenommen.

Die Messungen werden an der Produktinnenseite und/oder Produktaußenseite des Kennzeichnungsmittels aufweisenden Rückenbereichs vorgenommen.

Die Produktinnenseite versteht sich dabei als die für den Anwendungszustand körperzugewandte Oberseite und die Produktaußenseite als im Anwendungszustand körperabgewandte Oberseite des Inkontinenzartikels.

Die Messungen werden direkt am Produkt vorgenommen. Um die vom Kennzeichnungsmittel, bzw. vom ersten und/oder zweiten Kennzeichen in der Anwendung vermittelten Farbreize zu analysieren, werden die Farbmessungen an der Produktinnenseite und/oder Produktaußenseite im das Kennzeichnungsmittel aufweisenden Bereich und/oder in einem an das erste Kennzeichen direkt angrenzenden Bereich vorgenommen, unabhängig davon, ob die farbige Zone tatsächlich direkt auf einer die Produktinnenseite oder Produktaußenseite bildenden Materiallage, wie beispielsweise Oberseite des Deckblatts-Materials/Backsheet-Materials auf/angebracht ist oder nicht. Letzteren Falls wird auf die auf der Produktinnenseite oder Produktaußenseite erkennbaren ersten und zweiten Kennzeichen abgestellt.

Die zu vermessende Oberseite der Messprobe ist dabei dem Prüfgerät und dessen optischen Messfeldbereichen zugewandt angeordnet.

### Kalibrieren:

Das Prüfgerät ist nach den Herstellervorgaben und dafür vorgesehenen Standards kalibriert.

### Messvorgang:

Für die hierbei vorzunehmenden Messungen wird eine standardisierte weiße Testkarte als Referenzfläche unterhalb der Messprobe im Bereich des vorgesehenen Messfeldes gelegt, so dass die jeweiligen Farbmessungen stets gegenüber einer Standardfläche durchgeführt werden.

Als Referenzfläche werden die für die Opazitätsmessung vorgesehene "opacity charts" von BYK-Gardner, dabei die Karte #2810 eingesetzt (erhältlich bei BYK-Gardner GmbH, Geretsried, Deutschland).

Diese weiße Referenzflächen-Testkarte weist einen L*a*b*-Wert von
L* von 92,12 ± 0,79
a* von -0,95 ± 0,14
b* von 3,69 ± 0,42 auf,
dabei als Mittelwert ± 6 sigma (Standardabweichung)

Für den Messvorgang wird der Messkopf des Gerätes senkrecht und ohne zusätzlichen Druck auf die vorbereitete Messprobe und die gewünschte zu messende Oberseite der Messprobe aufgesetzt. Dafür wird, je nach Konstruktion des Prüfgerätes, zuerst mit einer Komponente die eine Messfeldbegrenzung aufweist (sogenannter Probenbeobachter), die für die Messung relevante Stelle der Messprobe auf der Messprobe festgelegt. Diesem Messfeld gegenüber wird dann das Prüfgerät mit seiner Messöffnung aufgesetzt.

Für das Prüfverfahren und den Messvorgang werden am Prüfgerät als Einstellungen vorgenommen:
- Skala: CIEL*a*b*
- Index: ΔE*
- Lichtart: D65
- Winkel: 10°
- Messöffnung: 11 mm
- im Falle von dazu schmaleren Messbereichen, ist ein Prüfgerät mit einer entsprechend schmäleren Messöffnung zu wählen

Es werden von einer Messprobe n = 3 Messungen vorgenommen.

Für die Farbmessung für das erste Kennzeichen ist vorrangig ein jeweiliger Bereich innerhalb der farbigen Zone 73 des ersten Kennzeichens innerhalb des Kennzeichnungsmittels heranzuziehen. Sollte kein genügend großer Bereich zur Verfügung zu stehen, so wird ein Messfeld mit dem größtmöglichen Anteil an erstem Kennzeichen, also an farbiger Zone 73 herangezogen.

Für die Farbmessung für das zweite Kennzeichen werden bei ausreichend großen Aussparungen 76 die Farbmessungen innerhalb der Aussparungen 76 vorgenommen. Andernfalls werden die Farbmessungen in einem außerhalb der Randumfangslinie 78 des ersten Kennzeichens direkt angrenzenden Bereich der die Außenseite und/oder Innenseite bildenden Materiallage, wie beispielsweise des Deckblattmaterials oder Backsheet-Materials, durchgeführt und zwar dort wo sich das die Außenseite bzw. Innenseite bildende Material, wie beispielsweise das Deckblattmaterial oder Backsheet-Material, visuell wahrnehmbar nicht von dem Material, wie beispielsweise vom Deckblattmaterial oder Backsheet-Material, in den Aussparungen unterscheidet. Dabei wird die Messung an verschiedenen Stellen durchgeführt.

### Auswertung:

Aus den Messwerten wird der Mittelwert ermittelt.

Die Messwerte und der Mittelwert werden auf eine Dezimale nach dem Komma angegeben. Die Farbtöne werden als L*, a*, b* Wert angegeben. Der Wert ist dimensionslos.

Für den Vergleich von Farbmessungen an verschiedenen Bereichen bzw. verschiedenen Messproben wird der Farbabstand bei Farbmessungen nach dem CIEL*a*b*-Farbraum als Delta-E (ΔE*) wie folgt berechnet:
- für die jeweilige Differenz von jeder der drei Achsen des CIEL*a*b*-Farbraums das Quadrat berechnen
- davon die Summe und über diese Summe die Wurzel berechnen:
$ΔE * = {\left[{\left(ΔL*\right)}^{2}+{\left(Δa*\right)}^{2}+{\left(Δb*\right)}^{2}\right]}^{0 , 5}$

Der Wert ist dimensionslos.

Dabei erfolgt die Messung der Opazität wie folgt:

### Opazität

Die Opazität ist ein Maß für die Lichtundurchlässigkeit einer Materialschicht, oder Materiallagen-Kombinationen und damit ein Maß für die Fähigkeit eines Materials, den Hintergrund dahinter zu verdecken. Die Opazität wird dabei bestimmt über das Verhältnis aus bei einem schwarzen Hintergrund für ein Material erhaltene Reflexionsvermögen und für das gleiche Material bei einem weißen Hintergrund erhaltene Reflexionsvermögen. Ein höherer Opazitätswert zeigt dabei einen höheren Grad an Lichtblockierung durch das Material an.

### Prüfgerät:

Für die Opazitätsmessungen wird als Prüfgerät ein Spektralphotometer eingesetzt, wie z.B. das Spectro-guide sphere gloss von BYK-Gardner.

### Kalibrieren:

Das Prüfgerät ist nach den Herstellervorgaben und dafür vorgesehenen Standards kalibriert.

### Messvorgang:

Für die Opazitätsmessung werden die vom Hersteller vorgegebenen Schwarz/Weiß-Opazitätskarten eingesetzt. Von den vorgesehenen "opacity charts" von BYK-Gardner, wird dabei die Karte #2810 eingesetzt (erhältlich bei BYK-Gardner GmbH, Geretsried, Deutschland).

Für das Prüfverfahren und den Messvorgang werden am Prüfgerät als Einstellungen vorgenommen:
- Skala: CIEL*a*b*
- Index: Opazität
- Lichtart: D65
- Winkel: 10°
- Messöffnung: 11 mm
- im Falle von dazu schmaleren Messbereichen, ist ein Prüfgerät mit einer entsprechend schmäleren Messöffnung zu wählen

Es werden von einer Messprobe n = 3 Messungen vorgenommen.

Für den Messvorgang wird der Messkopf des Gerätes senkrecht und ohne zusätzlichen Druck auf die vorbereitete Messprobe und die gewünschte zu messende Oberseite der Messprobe aufgesetzt. Es wird der Probenbeobachter verwendet um Einflüsse durch unterschiedliche Lichtbedingungen zu vermeiden. Die Messprobe sollte dabei das Messfeld des Prüfgerätes vollständig überdecken.

Die Opazität wird kann dabei im Differenzmodus zwischen der Messung über dem schwarzen Untergrund und der anschließenden Messung über weißem Untergrund bestimmt werden.

Das für die Opazität zu vermessende Material (Messprobe) wird dazu auf die S/W-Opazitätskarte gelegt: Dabei wird die Messprobe ohne Kontamination des vorgesehenen Messfeldes zunächst auf das schwarze Feld der S/W-Opazitätskarte gelegt. Der Messvorgang mittels des Prüfgerätes wird gestartet, die abgeschlossene Messung durch das Prüfgerät wird mit einem Signalton quittiert. Es erfolgt ein Abgleich nach dieser ersten Messung, indem die über schwarzem Untergrund gemessene Opazität auf Null gesetzt wird. Dann wird das gleiche Material, mit dem selben Probenausschnitt der vorangehenden Messung, auf das weiße Feld der S/W-Opazitätskarte positioniert, und dort wird ebenso die Messung mittels Prüfgerät durchgeführt. Der Messwert im weißen Feld wird bereits als Differenzwert ausgegeben und stellt den ermittelten Opazitätswert der Messprobe dar, mit der Angabe des Wertes in %.

Aus den Messwerten wird der Mittelwert ermittelt. Die Angabe erfolgt in Prozent.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen der erfindungsgemäßen Inkontinenzwegwerfwindel.

In der Zeichnung zeigen:
- Figur 1a: eine Draufsicht auf eine erfindungsgemäße Inkontinenzwegwerfwindel in einem flach gelegten und eben ausgebreiteten Zustand;
- Figur 1b: eine Gestaltung einer Inkontinenzwegwerfwindel ähnlich dem in Figur 1a Gezeigten zur Bestimmung eines Rückenendabschnitts und eines hinteren Quernahtabschnitts,
- Figur 2a: eine weitere Darstellung einer Inkontinenzwegwerfwindel,
- Figur 2b: eine weitere Darstellung einer Inkontinenzwegwerfwindel,
- Figuren 3a, 3b und 3c: eine Schnittdarstellung in Längsrichtung durch den Rückenendabschnitt der Inkontinenzwegwerfwindel in einer schematischen Darstellung,
- Figuren 4a, 4b und 4c: eine vergleichende Betrachtung eines mit dem Kennzeichnungsmittel im Rückenendabschnitt eingeschriebenen Bereichs mit einem analogen Bereich im Vorderendabschnitt,
- Figuren 5a, 5b und 5c: eine Inkontinenzwegwerfwindel mit vorgesehenen Faltungsabschnitten und Schnittdarstellungen von gefalteten Seitenabschnitten,
- Figur 6: schematisch eine vergrößerte Darstellung einer Ausführungsform des Kennzeichnungsmittels,
- Figuren 7a, 7b, 7c, 7d: das Kennzeichnungsmittel mit der Bestimmung eines inneren und eines äußeren Abstands ausgehend von der Randumfangslinie,
- Figuren 8a, 8b und 8c: eine Bestimmung des Anteils des ersten und zweiten Kennzeichens an der Fläche des Kennzeichnungsmittels,
- Figur 9: schematisch eine Bestimmung einer funktional zusammenhängenden Anordnung von Sub-Kennzeichnungsmittel zu einem Kennzeichnungsmittel und
- Figur 10: schematisch ein Kennzeichnungsmittel bestehend aus zwei Sub-Kennzeichnungsmitteln

Die Figuren zeigen eine insgesamt mit dem Bezugszeichen 2 bezeichnete wegwerfbare absorbierende Inkontinenzwegwerfwindel für die Aufnahme von im Wesentlichen flüssigen Körperausscheidungen.

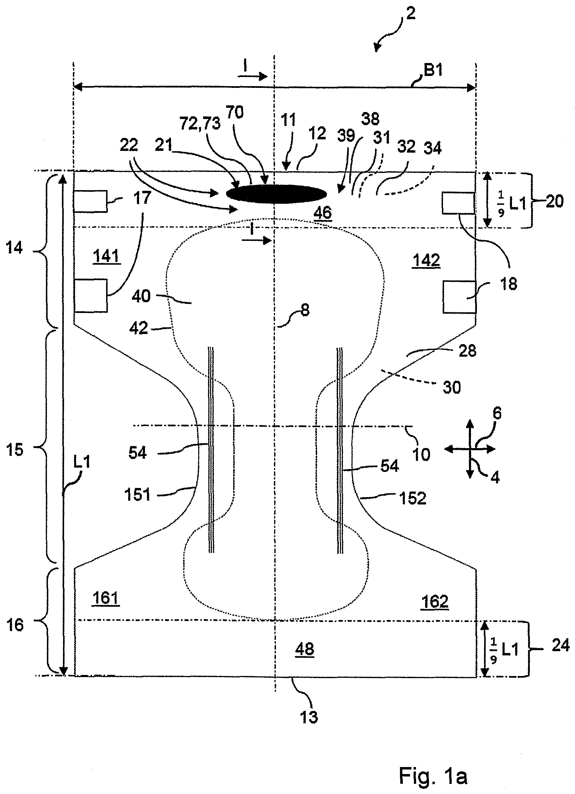

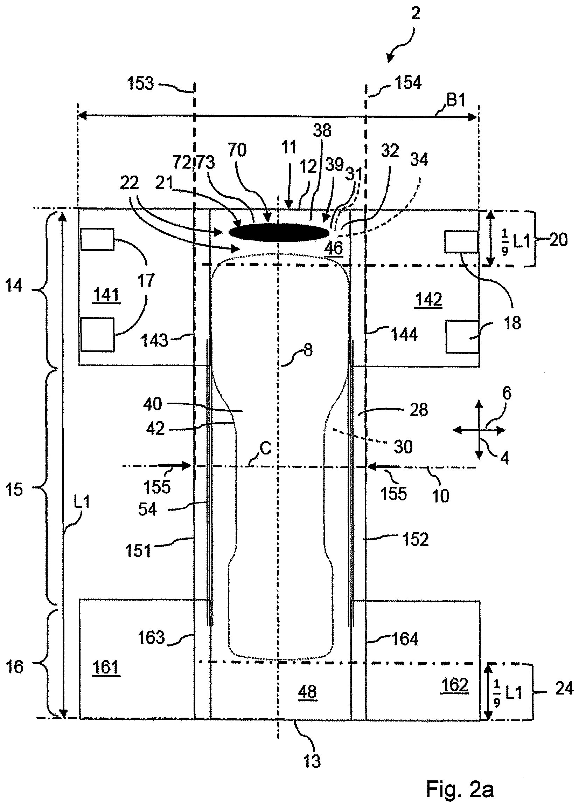

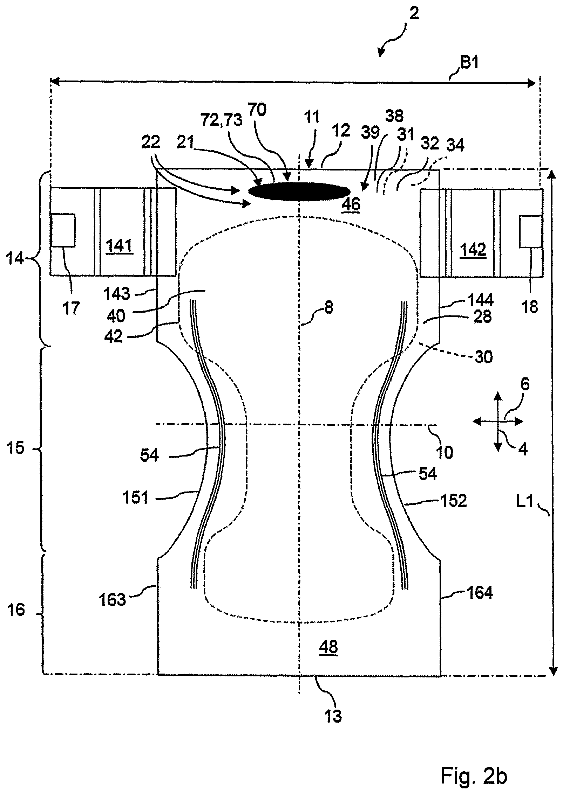

Die Inkontinenzwegwerfwindel 2 weist in Längsrichtung 4 über die Produktlänge L1 einen mit einem Saugkörper 40 aufweisenden Hauptteil 11 mit einem Rückenbereich 14, einem Vorderbereich 16 und einem in Längsrichtung 4 dazwischen liegenden und in der Anwendung zwischen den Beinen des Benutzers zu liegen kommenden Schrittbereich 15 auf. Im Rückenbereich 14 erstrecken sich hintere Seitenabschnitte 141, 142 in Querrichtung 6 zumindest über die seitlichen mittleren Längsränder 151,152 des Schrittbereichs 15 hinaus und weisen jeweils wenigstens ein Verschlussmittel 17, 18 auf, das an dem Vorderbereich 16 lösbar festlegbar ist und dadurch der Vorderbereich 16 und der Rückenbereich 14 miteinander verbindbar sind. Auch am Vorderbereich 16 können beidseits vordere Seitenabschnitte 161,162 vorgesehen sind, welche sich in Querrichtung 6 ebenso zumindest über mittlere seitliche Längsränder 151,152 des Schrittbereichs 15 hinaus erstrecken, wie in den Figuren 1a und 2a dargestellt ist. Die Seitenabschnitte können sich dabei integral aus dem Hauptteil erstrecken, wie eine bevorzugte Ausführungsform in Figur 1a zeigt, oder die Seitenabschnitte können als separate Komponenten an den Hauptteil angefügt sein, wie eine andere bevorzugte Ausführungsform in Figur 2a zeigt. Dabei sind bei einem Hauptteil 11 mit im Rückenbereich 14 hinteren seitlichen Längsrändern 143, 144, mit im Schrittbereich 15 mittleren seitlichen Längsrändern 151,152 und mit im Vorderbereich 16 vorderen seitlichen Längsrändern 163, 164 die jeweiligen hinteren Seitenabschnitte 141,142 und jeweiligen vorderen Seitenabschnitte 161,162 an den entsprechenden seitlichen hinteren 143,144 und seitlichen vorderen 163,164 Längsrändern des Hauptteils angefügt sind und erstrecken sich in Querrichtung 6 über die jeweiligen hinteren seitlichen 143,144 und/oder vorderen seitlichen Längsränder 163,164 des Hauptteils 11 hinaus. Es sind aber auch Inkontinenzwegwerfwindeln in einer weiteren bevorzugten Ausführung denkbar, die nur hintere seitliche Seitenabschnitte 141 142 aufweist, und bei der an den Vorderbereich keine Seitenabschnitte angefügt sind, sondern die vorderen seitlichen Längsränder des Hauptteils jeweils einen frei endenden Längsrand der Inkontinenzwegwerfwindel bilden, so wie schematisch in Figur 2b dargestellt ist.

Die Inkontinenzwegwerfwindel 2 mit einer Produktlänge L1 von vorzugsweise 500 - 1200 mm, vorzugsweise von 600 - 1000 mm und mit einer hinteren Produktspannbreite B1 von vorzugsweise 300 -1200 mm, vorzugsweise 400 -1100 mm, ist somit vor allem als eine Erwachsenen-Inkontinenzwegwerfwindel vorgesehen. Die hintere Produktspannbreite B1 umfasst dabei die Querstreckung des Hauptteils 11 zusammen mit den hinteren Seitenabschnitten 141, 142 in Querrichtung 6.

Die Inkontinenzwegwerfwindel 2 weist entlang ihrer Längsrichtung 4 unterschiedliche Eigenschaften auf. Eine mittels der Quermittelachse 10 abgrenzte hintere Produktlängshälfte und vordere Produktlängshälfte sind bezogen auf die Quermittelachse zumindest hinsichtlich einer Eigenschaft nicht spiegelsymmetrisch. Unterschiede können dabei insbesondere Eigenschaften, wie die Fläche des Produkts, die Fläche des Saugkörpers, insbesondere auch den Lagenaufbau des Saugkörpers, die Saugleistung des Saugkörpers und die chemische Zusammensetzung des Saugkörpers betreffen. Die damit verbundenen unterschiedlichen Funktionsbereiche innerhalb der Inkontinenzwegwerfwindel bedürfen somit der richtigen Anordnung der Inkontinenzwegwerfwindel am Benutzer.

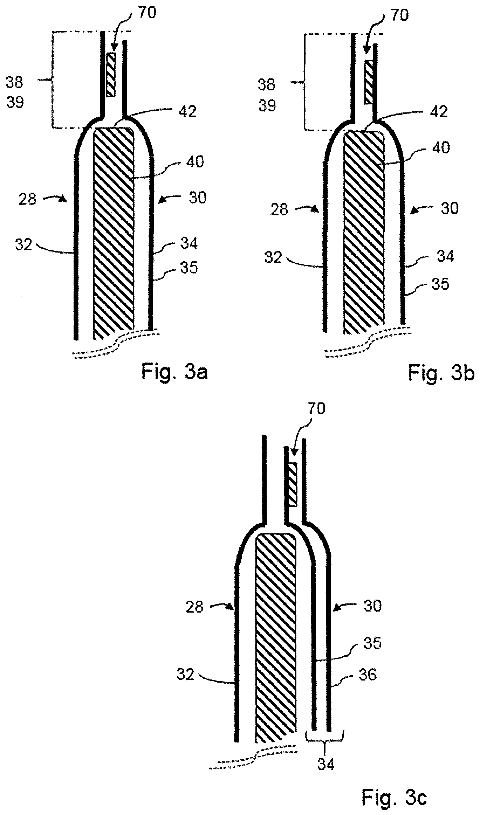

Die Inkontinenzwegwerfwindel 2 mit einer Innenseite 28, welche die im Gebrauch dem Körper des Benutzers zugewandte Oberseite dargestellt, und mit einer Außenseite 30, welche die im Gebrauch vom Körper des Benutzers abgewandte Oberseite darstellt, umfasst zumindest im Hauptteil 11 Chassismaterialien 31, wie insbesondere ein Deckblatt-Material 32 und ein Backsheet-Material 34, zwischen denen ein Saugkörper 40 angeordnet ist. In den vorliegenden Ausführungsbeispielen erstreckt sich das Deckblatt-Material 32 und das Backsheet-Material 34 in einen Bereich 39 außerhalb der den Saugkörper 40 begrenzenden Konturlinie 42 unter Ausbildung eines sogenannten Überhang 38, der schematisch in den Figuren 3a, 3b, 3c als Schnittansicht I -I der Figur 1a durch einen Rückenendabschnitt dargestellt ist.

Innerhalb eines Rückenendabschnitts 20 und innerhalb eines Vorderendabschnitt 24, die jeweils ein Neuntel (1/9) der Produktlänge L1 erfassen, ist ein hinterer Quernahtabschnitt 46 bzw. entsprechend ein vorderer Quernahtabschnitt 48 vorgesehen, wobei der Quernahtabschnitt den jeweiligen Bereich zwischen der den Saugkörper 40 begrenzenden Konturlinie 42 und den die Inkontinenzwegwerfwindel begrenzenden hinteren Hüftrand 12 bzw. vorderen Hüftrand 13 erfasst.

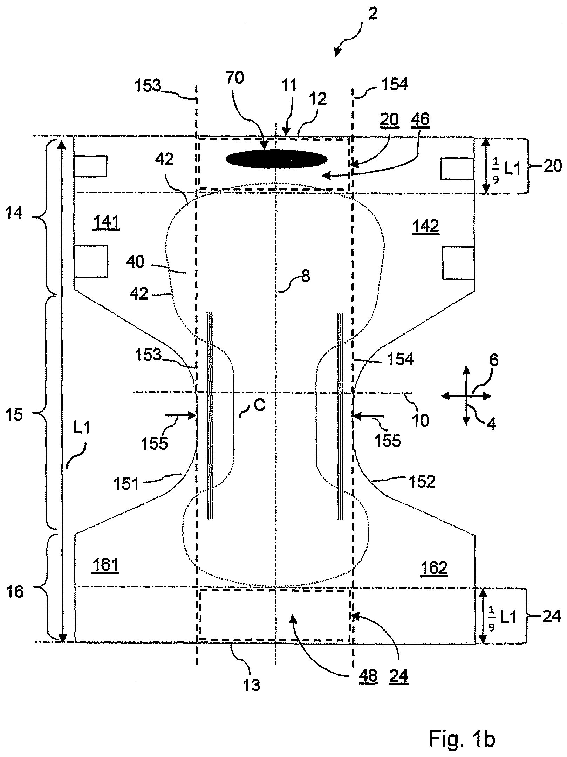

Figur 1b zeigt nun die Bestimmung eines Rückenendabschnitts 20 und eines hinteren Quernahtabschnitts 46 bzw. eines Vorderendabschnitts 24 und eines vorderen Quernahtabschnitts 48. Der Rückenendabschnitt 20 bzw. der Vorderendabschnitt 24 erfasst in Längsrichtung je ein Neuntel (1/9) der Produktlänge L1 ausgehend vom jeweiligen hinteren Hüftrand 12 bzw. vorderen Hüftrand 13. Die laterale Begrenzung des Rückenendabschnitts 20 und Vorderendabschnitts 24 wird durch eine jeweilige in Längsrichtung zur Längsmittelachse 8 parallel verlaufende Hilfslinie 153, 154 erhalten, die an den seitlichen mittleren Längsrand 151, 152 im Schrittabschnitt 15 an dessen schmalsten Stelle (C), mit den Pfeilen 155 markiert, tangierend angelegt und bis zum jeweiligen hinteren Hüftrand 12 bzw. vorderen Hüftrand 13 kreuzend weitergeführt wird. Innerhalb dieses begrenzten Rückenendabschnitts 20 bzw. Vorderendabschnitt 24 ist der hintere Quernahtabschnitt 46 bzw. vordere Quernahtabschnitt 48 angeordnet. Liegt hingegen keine schmalste Stelle innerhalb des Schrittabschnitts vor, so wird als Bezugspunkt zum Ansetzen der vorgenannten Hilfslinien 153 und 154 die an der Quermittelachse 10 vorhandene Querstreckung C des Hauptteils aufgenommen, wie in Figur 2a schematisch dargestellt ist und worin der Bezugspunkt 155 hierbei mit einem Pfeil markiert ist.

Die Inkontinenzwegwerfwindel weist zumeist beidseits in Längsrichtung 4 verlaufende Schrittelastifizierungsmittel 54 auf, die in Anwendung zur Ausbildung einer bootförmigen Konfiguration der Inkontinenzwegwerfwindel beitragen. Einwärts der Schrittleastifzierungsmittel 54 können auf der Innenseite 28 beidseits -hier nicht weiter dargestellte- in Längsrichtung 4 - erstreckende Cuffelemente vorgesehen sein, die ebenso mit Elastifizierungsmittel ausgestattet sind und eine Auslaufsperre für Flüssigkeiten darstellen.

Die Inkontinenzwegwerfwindel 2 weist, wie in Figur 1a, Figur 1b, Figur 2a und Figur 2b gezeigt, ein Kennzeichnungsmittel 70 auf, das im Rückenendabschnitt 20, dabei insbesondere vollumfänglich innerhalb des hinteren Quernahtabschnitts 46 angeordnet ist.

Der Rückenendabschnitt 20, insbesondere der hintere Quernahtabschnitt 46 umfasst zumindest einen ersten Bereich 21 und einen zweiten Bereich 22, wobei in dem ersten Bereich 21 das Kennzeichnungsmittel 70 vorgesehen ist. Das Kennzeichnungsmittel weist dabei ein erstes Kennzeichen 72 auf, das durch die flächenhaft erstreckte farbige Zone 73 gebildet ist und im vorliegenden Fall die Information transportiert, wo die Rückseite der Inkontinenzwegwerfwindel 2 ist. Darüber hinaus wird insbesondere durch die Wahl der Farbe eine Information darüber generiert, dass es sich um eine Inkontinenzwegwerfwindel von definierter Saugstärke handelt.

Im hinteren Quernahtabschnitt 46 sind das Deckblatt-Material 32 und das Backsheet-Material 34 vorzugsweise adhäsiv miteinander verbunden. Das Deckblatt-Material 32 umfasst vorzugsweise ein Vliesmaterial und das Backsheet-Material 34 umfasst zumindest eine Folienlage 35 und insbesondere bevorzugt zusätzlich eine zur Außenseite 30 angeordnete Vlieslage 36 unter Ausbildung eines sogenannten textilen Backsheets. Das Kennzeichnungsmittel 70 kann als Beschichtung auf einer der Außenseite 30 zugewandten Oberseite des Deckblatt-Materials 32 (Figur 3a) oder insbesondere auf einer der Innenseite 28 zugewandten Oberseite einer Folienlage 35 des Backsheet-Materials 34 (Figur 3b) angebracht sein. Bei einem textilen Backsheet (Figur 3c) ist bevorzugt auf der der Außenseite 30 zugewandten Oberseite der Folienlage 35 eine farbige Zone 73 vorgesehen, die das Kennzeichnungsmittel 70 bildet. Auf diese Weise wird erreicht, dass kein Abrieb der farbigen Zone erfolgen kann.

Die farbige Zone 73 des ersten Kennzeichens 72 des Kennzeichnungsmittel 70, wie in Figuren 1 und 2 dargestellt, ist hier in Form eines Farbaufdrucks auf eine der im hinteren Quernahtabschnitt 46 vorgesehenen Trägerflachbahnmaterialien, wie eben dem Chassismaterial 31, so dem Deckblatt-Material 32 oder dem Backsheet-Material 34 aufgebracht, dabei vorzugsweise in einer der Anordnung wie in den Figuren 3a - 3c gezeigt.

Das Kennzeichnungsmittel 70 ist dabei von der Innenseite 28 und von der Außenseite 30 der Inkontinenzwegwerfwindel visuell erkennbar.

Unterstützt wird die visuelle Erkennbarkeit des Kennzeichnungsmittels auf der Außenseite und Innenseite dadurch, dass zwischen dem Kennzeichnungsmittel und der jeweiligen Außenseite und Innenseite in dem ersten Bereich 21, in dem das Kennzeichnungsmittel angeordnet ist, vorzugsweise nur eine geringe Anzahl an Materiallagen vorgesehen ist, ganz besonders bevorzugt unterscheiden sich die Anzahl der Lagen zwischen Kennzeichnungsmittel zur Innenseite und der Anzahl der Lagen zwischen Kennzeichnungsmittel zur Außenseite höchstens um 2, insbesondere höchstens um 1 Lage.

In Figur 3a ist das Kennzeichnungsmittel 70 sowohl von der Innenseite 28 als auch von der Außenseite 30 von einer Flachbahnmateriallage, nämlich dem Deckblattmaterial 32 zur Innenseite und der Folie 35 zu Außenseite überdeckt. Entsprechendes mit nur einer Flachbahnmateriallage zwischen Kennzeichnungsmittel 70 und der Außenseite bzw. der Innenseite zeigt die Figur 3b. In Figur 3c ist das Kennzeichnungsmittel 70 ausgehend von der Innenseite 28 von der Lage des Deckblatt-Materials 32 und der Folienlage 35 des textilem Backsheet-Materials 34 überdeckt und ausgehend von der Außenseite 30 von der Vlieslage 36.

Das Deckblatt-Material, vorzugsweise aus Vliesstoffmaterial mit einem Flächengewicht von 5 - 20 g/m² und das Backsheet-Material mit einem Flächengewicht von vorzugsweise 9-30 g/m² weisen jeweils ein Opazität von höchstens 60%, vorzugsweise von höchstens 50%, insbesondere höchstens 40% auf, was die visuelle Erkennbarkeit des Kennzeichnungsmittels auf der Innenseite und Außenseite unterstützt.

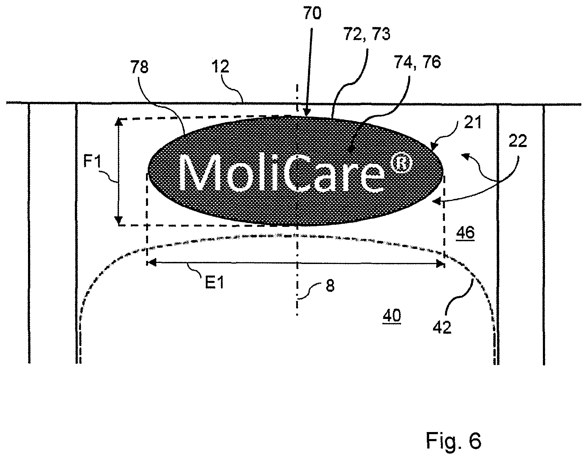

In einer Ausführungsform des Kennzeichnungsmittels 70, wie in Figur 6 vergrößert schematisch dargestellt ist, sind in dem Kennzeichnungsmittel 70 bzw. in der farbigen Zone 73 des ersten Kennzeichens 72 Ausnehmungen 76 vorgesehen, die mit einem gewissen Abstand zur Randumfangslinie 78 des Kennzeichnungsmittels 70 angeordnet sind.

Die Ausnehmungen 76 bilden ein zweites Kennzeichen 74, das eine weitere Information transportiert, nämlich vorzugweise über die Vorsehung von alphanumerischen Zeichen, den Hinweis über einen Hersteller bzw. seiner Marke.

Mit der Ausgestaltung der Ausnehmungen 76 als alphanumerische Zeichen, dessen informativen Inhalt und Kontext der Anwender durch intuitive Lesen erfasst, wird die Aufmerksamkeit eines Anwenders noch mehr auf das Kennzeichnungsmittel 70 gelenkt und damit die Wahrnehmungskraft des Kennzeichnungsmittels unterstützt.

Dabei bilden die Aussparungen 76 Bereiche in der farbigen Zone 73, in denen insbesondere die visuell wahrnehmbare Beschaffenheit des Materials derjenigen visuell wahrnehmbaren Beschaffenheit des Materials des zweiten Bereichs 22 im hinteren Quernahtabschnitt 46 identisch ist. Auf diese Weise kann eine dezente und doch gut lesbare zweite Information geliefert werden. Insbesondere durch das Einschließen in eine farbige Zone 73 wird die zweite Information des zweiten Kennzeichens 74 besser wahrnehmbar, als wenn eine herkömmliche Ausgestaltung des zweiten Kennzeichens in Form einer herkömmlichen Bedruckung ohne den umschließenden Bereich des ersten Kennzeichens vorgesehen wäre. Durch den sich visuell vom zweiten Bereich 22 des Materials unterscheidbaren abhebenden ersten Bereich 21 des ersten Kennzeichens 72 wird ein Kontrast geschaffen, der die vergleichsweise kleinere schlechter erkennbare Information des zweiten Kennzeichens bezüglich der Wahrnehmbarkeit verbessert.

Das Kennzeichnungsmittel 70, vorzugsweise in der Ausführung eines Einzelelements, weist in Querrichtung eine maximale Quererstreckung E1 und in Längsrichtung eine maximale Längserstreckung F1 auf. Dabei ist insbesondere vorgesehen, dass die maximale Querstreckung E1 des Kennzeichnungsmittels in Querrichtung der Inkontinenzwegwerfwindel größer ist als eine maximale Längserstreckung F1 des Kennzeichnungsmittels in Längsrichtung der Inkontinenzwegwerfwindel. Bei der Handhabung einer an sich ohnehin länglich erscheinenden Inkontinenzwegwerfwindel 2 wirkt ein Kennzeichnungsmittel 70 mit einer in Querrichtung der Inkontinenzwegwerfwindel ausgerichteten vergleichsweise größeren maximalen Querstreckung E1 als dessen Längserstreckung F1 in Längsrichtung der Inkontinenzwegwerfwindel prägnanter und ihm kommt damit eine größere Wahrnehmungskraft zu.

Der Wahrnehmung des Kennzeichnungsmittels innerhalb des Rückenendabschnitts ist es insbesondere zuträglich, wenn bei Betrachtung der Inkontinenzwegwerfwindel auf der Innenseite oder bei Betrachtung der Inkontinenzwegwerfwindel auf der Außenseite ein Unterschied an der Ausgestaltung innerhalb des Rückenendabschnitts und Vorderendabschnitts vorliegt, indem der Vorderendabschnitt entweder frei ist von farbiger Markierung oder im Falle einer farbigen Markierung dann eine für den Anwender unterscheidbare weniger prägnante Ausgestaltung der farbigen Markierungen im Vorderendabschnitt gegeben ist im Vergleich zur farbigen Zone des Kennzeichnungsmittels im Rückenendabschnitt.

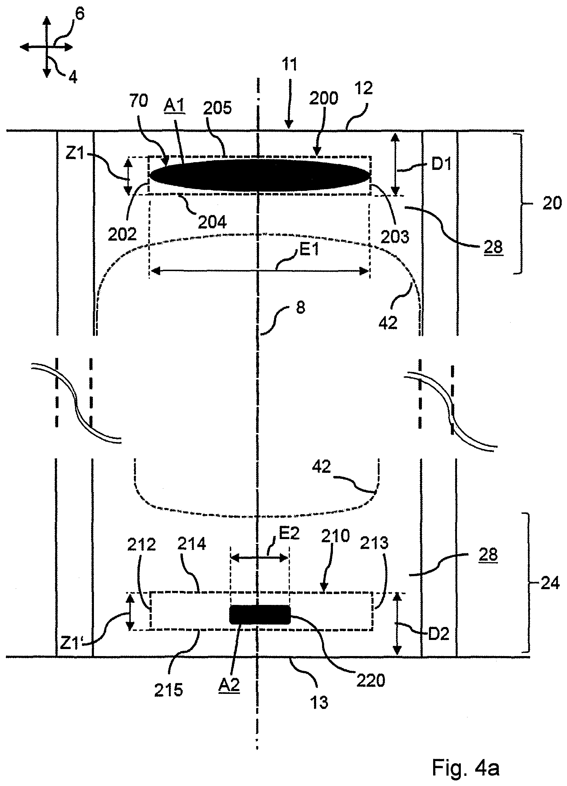

Die Figur 4a zeigt dazu eine vergleichende Betrachtung des im Rückenendabschnitt 20 das Kennzeichnungsmittel 70 aufweisenden Bereichs mit einem entsprechenden in Längsrichtung 6 im Vorderendabschnitt 24 verorteten Bereich.

Dabei wird der Vergleich der Bereiche innerhalb jeweils einer Oberseite, d.h. auf der Innenseite oder auf der Außenseite durchgeführt. Figur 4a zeigt die Aufsicht auf die Innenseite 28 im jeweiligen Ausschnitt des Rückenendabschnitts 20 und des Vorderendabschnitts 24.

Hierzu wird das Kennzeichnungsmittel 70, das eine Fläche A1 mit einer maximalen Quererstreckung E1 überfängt, von einem ersten imaginären Rechteck 200 gerade einbeschrieben ist, wobei das erste imaginäre Rechteck 200 erste und zweite Längsseiten 202, 203 parallel zu der Längsrichtung 4 und eine erste innere Querseite 204 und eine erste äußere Querseite 205 parallel zur Querrichtung 6 aufweist. Die erste und zweite Längsseite 204, 205 weisen die Länge Z1 auf und die erste innere Querseite 204 einen ersten Abstand D1 vom hinteren Hüftrand 12.

In dem Vorderendabschnitt 24 wird auf derselben Innenseite 28 ein zweites imaginäres Rechteck 210 verortet, wobei das zweite imaginäre Rechteck 210 im Vergleich zum ersten imaginären Rechteck 200 parallel zur der Längsmittelachse 8 verschoben ist. Dabei weist das zweite imaginäre Rechteck 210 eine dritte und eine vierte Längsseite 212, 213 parallel zu der Längsrichtung 4 auf, und eine zweite innere Querseite 214 und zweite äußere Querseite 215 parallel zu der Querrichtung 6. Die dritte und vierte Längsseite 212, 213 weisen eine Länge Z1' auf, wobei die Länge Z1' der Länge Z1 des ersten imaginären Rechtecks 200 entspricht (Z1'=Z1). Die zweite innere Querseite 214 weist einen Abstand D2 vom vorderen Hüftrand 13 auf.

Für den Abstand D2 ist dabei eine Varianz derart vorgegeben, so dass gilt:
a) D2 ≤ 1,5x D1 und
b) die zweite äußere Querseite 215 des zweiten imaginären Rechtecks 210 darf sich nicht über den vorderen Hüftrand 13 hinauserstrecken.

In Figur 4a ist das zweite imaginäre Rechteck 210 innerhalb des Vorderendabschnitts 24 mit einem Abstand D2 verortet, wobei D2 dem Abstand D1 entspricht (D2 = D1).

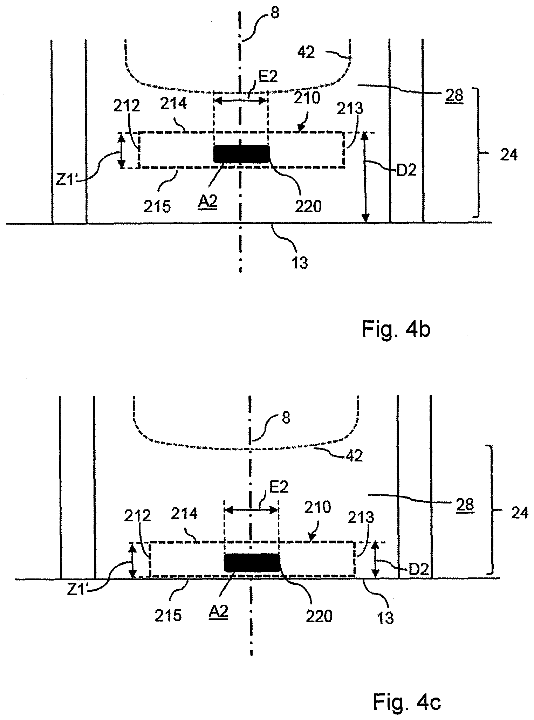

In den Figuren 4b und 4c sind die vorangehend genannten Varianzen des Abstandes D2, also die Grenzen innerhalb derer das zweite imaginäre Rechteck hinsichtlich des Abstandes D2 verortbar ist, schematisch innerhalb des Vorderendabschnitts 24 dargestellt. So zeigt Figur 4b die Verortung des zweiten imaginären Rechtecks 210, bei der der Abstand D2 das eineinhalbfache des Abstandes D1 (D2 = 1,5 x D1) beträgt. Figur 4c zeigt die Anordnung des zweiten imaginären Rechtecks 210 derart, dass sich die zweite äußere Querkante 215 sich nicht über den vorderen Hüftrand 13 hinauserstreckt, sondern D2 der Kantenlänge Z1 bzw. Z1' entspricht.

Für jede der Positionen, in die das zweite imaginäre Rechteck 210 verortet ist, ist vorzugsweise innerhalb des zweiten imaginären Rechtecks 210 keine visuelle erkennbare Markierung vorhanden.

Im Falle, dass eine visuell erkennbare Markierung 220 vorhanden ist, wie in den Figuren 4a-4c dargestellt, so ist die visuelle erkennbare Markierung 220 in ihrer Fläche A2 und/oder in ihrer maximalen Querstreckung E2 begrenzt. Die visuelle Markierung 220 weist eine Fläche A2 von höchstens 25% der Fläche A1 des Kennzeichnungsmittels 70 auf und/oder weist eine maximale Querstreckung E2 von höchstens 40% der maximalen Quererstreckung E1 des Kennzeichnungsmittels 70 auf.

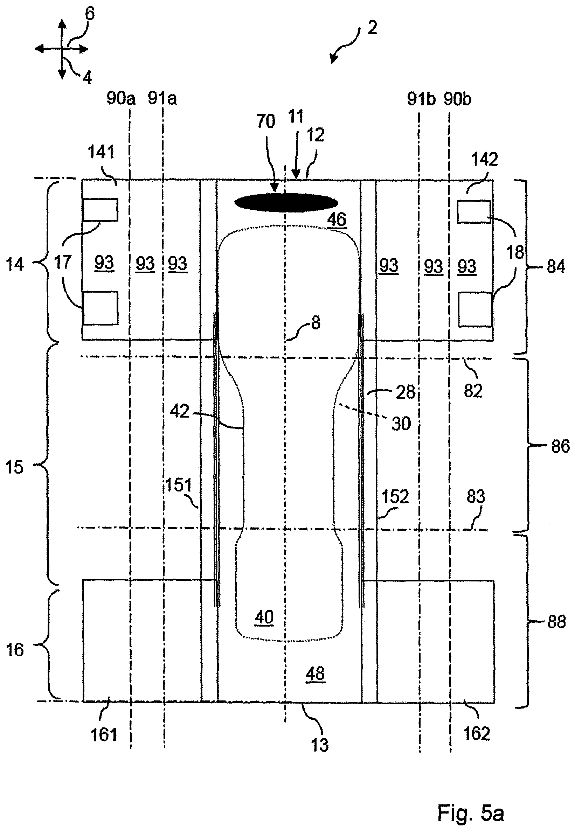

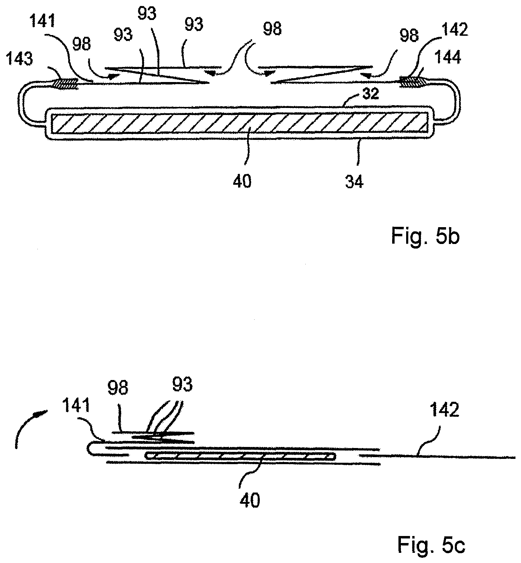

Für den Vertrieb der Inkontinenzwegwerfwindel 2 ist diese zumeist herstellerseitig in eine gefaltete Konfiguration verbracht. Figur 5a zeigt die Inkontinenzwegwerfwindel 2 von der Innenseite 28 vor der Überführung in eine gefaltete Konfiguration. So können die hinteren Seitenabschnitte 141, 142 und die vorderen Seitenabschnitte 161, 162 herstellerseitig wenigstens um eine in Längsrichtung 4 verlaufende Faltachse 90a, 90b, 91a, 91b auf die Innenseite und/oder auf sich selbst gefaltet sein. In einer bevorzugten Ausführung mit wenigstens 2 Faltachsen können die innerhalb der Seitenabschnitte erhaltenen Seitenabschnittssegmente 93 dabei aufeinander abgelegt sein, wie in einer Schnittansicht in der -Figur 5 b gezeigt ist, und auch noch entsprechende Einfass- und Anfassbereiche 98 verbleiben. Bei 3 Faltachsen innerhalb der Seitenabschnitte können die Seitenabschnittsegmente 93, wie in den Figur 5c gezeigt ist, abgelegt werden.

Mit Faltung der Seitenabschnitte wird die Inkontinenzwegwerfwindel in eine die ursprüngliche Produktspannbreite reduzierende Zwischenkonfiguration überführt. Um auch in Längsrichtung die Inkontinenzwegwerfwindel zu einer vertriebstauglichen Konfiguration zu minimieren ist eine Faltung um mindestens eine, vorzugsweise um zwei in Querrichtung 6 verlaufende Querfaltachsen 82,83 vorgesehen unter Erhalt mit einem in Längsrichtung 4 vorgesehenen hinteren Faltungsabschnitt 84, einem mittleren Faltungsabschnitt 86 und einem vorderen Faltungsabschnitt 88, wie in Figur 5a dargestellt. Das Kennzeichnungsmittel 70 im Rückenendabschnitt ist innerhalb des hinteren Faltungsabschnitts 84 angeordnet.

Bei Faltung um die in Querrichtung 6 verlaufenden Faltachsen 82, 83 kann dabei zuerst der hintere Faltungsabschnitt 84 um die Querfaltachse 82 einwärts auf die Innenseite 28 gefaltet werden oder zuerst der vordere Faltungsabschnitt 88 um die Querfaltachse 83 einwärts auf die Innenseite 28. Dem folgend wird der entsprechend andere vordere Faltungsabschnitt 88 bzw. hintere Faltungsabschnitt 84 um die jeweils andere Querfaltachse auf die Zwischenkonfiguration aufgefaltet.

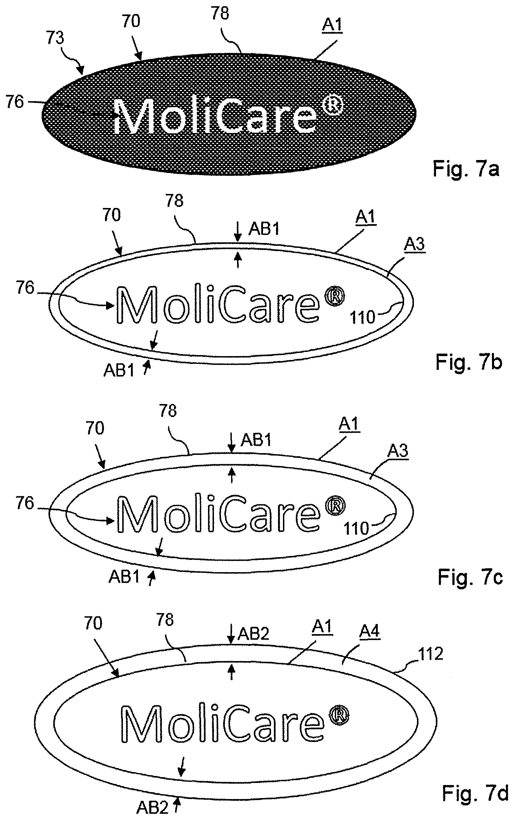

Für die Attraktion des Kennzeichnungsmittels ist eine gewisse flächenhafte Darstellung des ersten Kennzeichens wesentlich, insbesondere betrachtet ausgehend von einer Randumfangslinie 78 des Kennzeichnungsmittels. Ebenso vorteilhaft wird die Attraktion des Kennzeichnungsmittels bzw. damit auch des ersten Kennzeichens durch eine weitgehend solitäre Darstellung erreicht, also in einem äußeren Abstand von der Randumfangslinie 78 weitgehend frei von farbigen Zonen, insbesondere frei von weiteren farbigen Beschichtungen. Dies wird nachfolgend anhand Figuren 7a bis 7d erläutert.

Figuren 7a - 7d zeigen das Kennzeichnungsmittel 70, wobei in Figur 7a zu illustrativen Zwecken auf die Darstellung unten näher beschriebener imaginärer Abstandslinien verzichtet wurde und in den Figuren 7b - 7d zu illustrativen Zwecken die Darstellung der farbigen Zone 73 weggelassen wurde.

Die Fläche des Kennzeichnungsmittels 70, mithin die durch die Randumfangslinie 78 des ersten Kennzeichnungsmittels 70 einbeschriebene Fläche ist mit A1 bezeichnet. A3 (Figuren 7b und 7 c) bezeichnet die Fläche, welche von der Randumfangslinie 78 und einer ersten imaginären Abstandslinie 110 begrenzt wird, wobei die imaginäre Abstandslinie 110 innerhalb der Fläche A1 und in einem konstanten ersten Abstand AB1 von 2 mm nach innen beabstandet von der Randumfangslinie 78 verläuft. Die Größe der Fläche A3, gemessen in mm², beträgt im Falle der Figur 7b 20% der Größe der Fläche A1. In Figur 7c ist die imaginäre Anstandslinie 110 in größerem ersten Abstand AB1 von 3 mm von der Randumfangslinie 78 des ersten Kennzeichnungsmittels 70 skizziert. Die Größe der Fläche A3 beträgt hier 30% der Größe der Fläche A1.

Die Fläche A3 sollte im Wesentlichen durch das erste Kennzeichen gebildet und ausgefüllt sein; der Flächenanteil des zweiten Kennzeichens (also der Aussparungen 76), an der Größe der Fläche A3 beträgt vorteilhaft höchstens 10%, weiter insbesondere höchstens 7%, weiter insbesondere höchstens 5%.

In Figur 7d bezeichnet A4 die Fläche, welche von der Randumfangslinie 78 des Kennzeichnungsmittels 70 und einer zweiten imaginären Abstandslinie 112 begrenzt wird, wobei die zweite imaginäre Abstandslinie 112 außerhalb der Fläche A1 und in einem konstanten zweiten Abstand AB2 von 5 mm beabstandet von der Randumfangslinie 78 verläuft. Die Größe der Fläche A4, gemessen in mm², beträgt hier in etwa 50% der Größe der Fläche A1. Die Fläche A4 ist im Wesentlichen, insbesondere zu mindestens 90%, insbesondere zu 100% frei von weiteren farbigen Zonen, insbesondere frei von farbigen Beschichtungen und/oder Bedruckungen.

Figuren 8a - 8c zeigen nun in einzelnen Schritten eine Bestimmung des Anteils des zweiten Kennzeichens und dessen Verteilung an der Fläche des Kennzeichnungsmittels 70. Hierzu wird das Kennzeichnungsmittel 70 (Figur 8a) durch ein Rechteck 100 tangierend umschlossen (Fig. 8b), das bezüglich seiner Kanten in Längs- und Querrichtung 4 bzw. 6 der Inkontinenzwegwerfwindel 2 verläuft. Mit dem Rechteck 100 werden die jeweils äußersten Punkte des Kennzeichnungsmittels in dessen Quer- und Längsstreckung gerade einbeschrieben. Es erfolgt dann eine Teilung in gleich große Unterrechtecke des Rechtecks 100, die hier mit 101 bezeichnet sind, wobei sowohl in Längs- als auch in Querrichtung 4, 6 jeweils zehn Unterrechtecke 101 vorgesehen sind. Es wird anschließend die Anzahl N1 an Unterrechtecken 101a ermittelt, welche vollständig oder zumindest bereichsweise innerhalb der Randumfangslinie 78 des Kennzeichnungsmittels 70 lokalisiert sind. Es wird dann weiter innerhalb dieser die Anzahl N1 der Unterrechtecke 101a die Anzahl N2 derjenigen Unterrechtecke 101b bestimmt (in Figur 8c), welche bereichsweise sowohl das erste Kennzeichen als auch das zweite Kennzeichen aufweisen. Im vorliegenden Fall sind N1 = 88 Unterrechtecke 101a vorhanden, davon sind N2 = 27 Unterrechtecke 101b sowohl mit dem ersten Kennzeichen als auch mit dem zweiten Kennzeichen versehen. Diese 27

Unterrechtecke 101b sind in Figur 8c zur Veranschaulichung dunkel unterlegt. Somit liegt ein Verteilungsgrad von N2/N1*100% von 31 % vor.

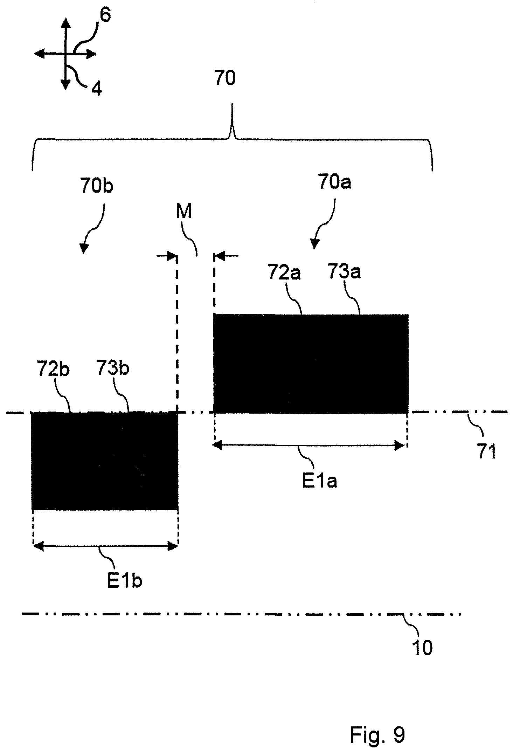

Das Kennzeichnungsmittel 70 kann alternativ zu dem in den vorangehenden Figuren Gezeigtem, wobei das Kennzeichnungsmittel als ein Einzelelement ausgebildet, auch eine Anordnung von zwei Sub-Kennzeichnungsmittel 70a, 70b, weiter insbesondere eine Vielzahl an Sub-Kennzeichnungsmittel aufweisen. Die funktionale Verbundenheit von mehreren Sub-Kennzeichnungsmittel 70a, 70b zu einem Kennzeichnungsmittel 70 ist dadurch gegeben, wie in Figur 9 schematisch dargestellt ist,
- dass die von zwei Sub-Kennzeichnungsmittel 70a, 70b benachbarten farbigen Zonen 73a, 73b der jeweiligen ersten Kennzeichen 72a, 72b auf einer parallel zur Quermittelachse 10 in Querrichtung 6 der Inkontinenzwegwerfwindel verlaufenden Linie 71 angeordnet sind und
- dass bei einem ersten Sub-Kennzeichnungsmittel 70a mit einer maximalen Quererstreckung E1a und bei einem benachbarten zweiten Sub-Kennzeichnungsmittel 70b mit einer maximalen Querstreckung E1b, der Abstand M zwischen den beiden Sub-Kennzeichnungsmittel 70 a, 70b als die kürzeste Distanz in Querrichtung 6 zwischen den benachbarten farbigen Zonen höchstens 25 %, insbesondere höchstens 20%, insbesondere höchstens 15%, insbesondere höchstens 10% der ersten oder zweiten maximalen Quererstreckung E1a oder E1b beträgt, dabei im Falle von unterscheidbaren ersten und zweiten Querstreckung E1a und E1b auf die kleinere maximale Quererstreckung von beiden maximalen Querstreckung E1a und E1b bezogen ist.

"Auf einer zur Quermittelachse parallelen Linie angeordnet" versteht sich als Positionierung der farbigen Zone, bei der diese Linie die farbige Zone in ihrer flächenhaften Erstreckung zumindest tangiert, vorzugsweise durchkreuzt.

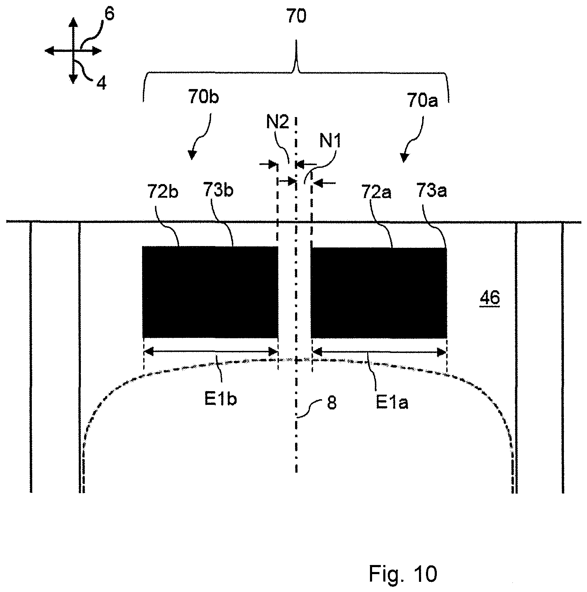

Insbesondere kann ein an der Längsmittelachse 8 angeordnetes Kennzeichnungsmittel 70 eine Anordnung aus zwei Sub-Kennzeichnungsmittel 70a, 70b mit je einem ersten Kennzeichen 72a, 72b aufweisen, wie schematisch in Figur 10 dargestellt ist.

Die zwei Sub-Kennzeichnungsmittel 70a 70 b gleichen sich dabei insbesondere hinsichtlich eines Abstandes N1, N2 des einzelnen Sub-Kennzeichnungsmittels zur Längsmittelachse und/oder insbesondere hinsichtlich einer maximale Querstreckung E1a, E1b des jeweiligen Sub-Kennzeichnungsmittels und /oder insbesondere hinsichtlich einer vom jeweiligen Sub-Kennzeichnungsmittel eingeschriebenen Fläche und/oder insbesondere hinsichtlich einer vom ersten Kennzeichen eingeschriebenen Fläche und Kontur, also Form der farbigen Zone 73a, 73 b. Insbesondere sind die zwei Sub-Kennzeichnungsmittel im Wesentlichen zueinander symmetrisch an der Längsmittelachse angeordnet.

Der jeweilige Abstand N1, N2 des einzelnen Sub-Kennzeichnungsmittels zur Längsmittelachse beträgt höchstens 20%, insbesondere höchstens 15%, insbesondere höchstens 10% der maximalen Querstreckung des Sub-Kennzeichnungsmittels.

Das erste Kennzeichen eines Kennzeichnungsmittels, gebildet durch eine flächenhaft erstreckte farbige Zone in einem ersten Bereich des Rückenbereichs der Inkontinenzwegwerfwindel, unterscheidet sich bei Aufsicht auf die Produktaußenseite und bei Aufsicht auf die Produktinnenseite von dem das erste Kennzeichen direkt umgebenden zweiten Bereich vorteilhafterweise in den nach CIEL*a*b* zueinander ermittelten Farbabständen, dargestellt als ΔE*. Vorzugsweise beträgt der Farbstand 15 - 55. Damit kommt dem Kennzeichnungsmittel eine gute Signalwirkung zu.

In der bevorzugten Ausführungsform des Kennzeichnungsmittels 70 mit einem innerhalb des ersten Kennzeichens durch Aussparungen erhaltenen zweiten Kennzeichens schaffen nach CIEL*a*b* ermittelte Farbabstände von vorzugsweise 15 - 55 einen Kontrast zwischen dem ersten und zweiten Kennzeichen, was die Wahrnehmbarkeit des zweiten Kennzeichens verbessert.

Beispielhaft ist das Kennzeichnungsmittel 70 in einem hinteren Quernahtabschnitt 46 mit einem Lagenaufbau, wie schematisch in Figur 3c gezeigt, umfassend ein Deckblatt-Material 32 aus Vliesmaterial und ein textiles Backsheet-Material 34 mit einer inneren Folienlage 35 und einer äußeren Vlieslage 36 auf der der Außenseite 30 zugewandten Oberseite der inneren Folienlage angebracht. Dabei ist die flächenhafte erstreckte farbige Zone 73 des ersten Kennzeichens 72 bei einer ersten Inkontinenzwegwerfwindel mit einer blau farbenen Druckfarbe eingebracht, bei einer zweiten Inkontinenzwegwerfwindel mit einer grün farbenen Druckfarbe.

Das Deckblatt-Material aus einem PP-Spinnvlies weist ein Flächengewicht von 12-14 g/m² und eine Opazität von 22- 26% auf, das texile Backsheet-Material mit einer PE-Folie und einem PP-Spinnvlies weist ein Flächengewicht von 18 g/m² und eine Opazität von 50-60% auf.

Das erste und zweite Kennzeichen eines Kennzeichnungsmittels und der das Kennzeichnungsmittel direkt umgebende zweite Bereich unterscheiden sich bei Aufsicht auf das Produkt von der Außenseite und der Innenseite in deren Farben, gemessen nach CIEL*a*b* und den zueinander ermittelten Farbabständen, dargestellt als ein jeweiliges ΔE*. In Tabelle 1 und 2 sind dazu die Ergebnisse dargestellt.

**Tabelle 1: Beispiele für Ausführungsformen mit erstem und zweitem Kennzeichen und deren Farbbestimmung nach CIEL*a*b***

| Windel 1 | Kennzeichen 1 Blau P540 | | | Kennzeichen 2 / direkt umgebender Bereich | | |
|---|---|---|---|---|---|---|
| | *L | *a | *b | *L | *a | *b |
| Produktaußenseite | 49,61 | 2,13 | -11,42 | 92,96 | -0,68 | 2,05 |
| Produktinnenseite | 62,96 | -1,27 | -9,35 | 92,46 | -0,71 | 2,32 |
| | | | | | | |

| Windel 2 | Kennzeichen 1 Grün P3282 | | | Kennzeichen 2 / direkt umgebender Bereich | | |
|---|---|---|---|---|---|---|
| | *L | *a | *b | *L | *a | *b |
| Produktaußenseite | 60,25 | -22,21 | -2,14 | 92,96 | -0,68 | 2,05 |
| Produktinnenseite | 67,50 | -12,85 | -4,51 | 92,46 | -0,71 | 2,32 |

**Tabelle 2: Farbabstände von erstem Kennzeichen zu zweitem Kennzeichen bzw. direkt umgebenden Bereich, dargestellt als Delta E und daraus ermittelte Unterschiede**

| | Farbabstand | Windel 1 (blaues Kennzeichen 1) | Windel 2 (grünes Kennzeichen 1) |
|---|---|---|---|
| Produktaußenseite | Delta E (Δ E*) | 45,5 | 39,4 |
| Produktinnenseite | Delta E (Δ E*) | 31,7 | 28,6 |
| | | | |
| Differenz Delta E (Δ E*) Produktaußenseite - Produktinnenseite | | 13,76 | 10,80 |

## Patentansprüche

1. Absorbierende Inkontinenzwegwerfwindel (2) mit einer Längsrichtung (4) und einer Querrichtung (6) , mit einer Längsmittelachse (8) und einer Quermittelachse (10), mit einer Innenseite (28) und einer Außenseite (30), mit einem einen Saugkörper (40 ) aufweisenden Hauptteil (11) mit einem hinteren Hüftrand (12) und einem vorderen Hüftrand (13), umfassend einen Rückenbereich (14), einen Vorderbereich (16), und einen in Längsrichtung (4 ) dazwischen liegenden, zwischen den Beinen eines Benutzers zu liegen kommenden Schrittbereich (15), und mit beidseits im Rückenbereich (14) vorgesehenen hinteren Seitenabschnitte (141, 142), welche sich in Querrichtung (6) zumindest über seitliche mittlere Längsränder (151,152) des Schrittbereichs (15) hinaus erstrecken und jeweils wenigstens ein Verschlussmittel (17,18) aufweisen, das an dem Vorderbereich (16) lösbar festlegbar ist und dadurch der Vorderbereich (16) und der Rückenbereich (14) miteinander verbindbar sind, wobei im Rückenbereich (14) ein an der Längsmittelachse (8) angeordnetes Kennzeichnungsmittel (70) vorgesehen ist, das zumindest bereichsweise sowohl auf der Innenseite (28) als auch auf der Außenseite (30) visuell erkennbar ist, wobei das Kennzeichnungsmittel (70) in einem ersten Bereich (21) des Rückenbereichs (14) ein erstes Kennzeichen (72) aufweist, das durch eine flächenhaft erstreckte farbige Zone (73) gebildet ist und das eine erste Information bereitstellt, wobei die farbige Zone (73) visuell unterscheidbar ist von einem das erste Kennzeichen (72) umgebenden zweiten Bereich (22) des Rückenbereichs (14) **dadurch gekennzeichnet, dass** das Kennzeichnungsmittel (70) ein zweites Kennzeichen (74) umfasst, das eine zweite Information bereitstellt, das dadurch gebildet ist, dass in dem ersten Kennzeichen (72) Aussparungen (76) der farbigen Zone (73) vorgesehen sind.

2. Inkontinenzwegwerfwindel nach Anspruch 1, **dadurch gekennzeichnet, dass** das Kennzeichnungsmittel (70) in einem Rückenendabschnitt (20) vorgesehen ist, insbesondere vollständig in einem Rückenendabschnitt (20) vorgesehen ist und/oder dass im Rückenbereich (14) zwischen einer den Saugkörper (40) begrenzenden Konturlinie (42) und dem hinteren Hüftrand (12) ein hinterer Quernahtabschnitt (46) vorgesehen ist, in dem das Kennzeichnungsmittel (70) zumindest bereichsweise angeordnet ist, vorzugsweise vollständig angeordnet ist, wobei insbesondere der hintere Quernahtabschnitt (46) einen Überhang (38) eines den Saugkörper (40) umgebenden Chassismaterials (31), insbesondere eines Deckblatt-Materials (32) und/oder eines Backsheet-Materials (34) umfasst oder daraus gebildet ist, in einem Bereich (39) in dem das Chassismaterial (31), insbesondere das Deckblatt-Material (32) und/oder das Backsheet-Material (34) sich außerhalb einer den Saugkörper (40) begrenzenden Konturlinie (42) erstreckt.

3. Inkontinenzwegwerfwindel nach einem der vorherigen Ansprüche, **dadurch gekennzeichnet, dass** das Kennzeichnungsmittel (70) ein Einzelelement ist.

4. Inkontinenzwegwerfwindel nach Anspruch 1, **dadurch gekennzeichnet, dass** das zweite Kennzeichen (74) alphanummerische Zeichen umfasst.

5. Inkontinenzwegwerfwindel nach einem der vorherigen Ansprüche, **dadurch gekennzeichnet, dass** das Kennzeichnungsmittel (70) auf einem Backsheet-Material (34), dabei auf einer der Innenseite (28) zugewandten Oberseite oder einer der Außenseite (30) zugewandten Oberseite des Backsheet-Materials angebracht ist, oder insbesondere bei einem Backsheet-Material umfassend eine innere Folienlage (35) und eine äußere Vlieslage (36) das Kennzeichnungsmittel (70) dazwischen und dabei insbesondere auf der der Außenseite (30) zugewandten Oberseite der Folienlage (35) angebracht ist oder dass das Kennzeichnungsmittel (70) auf einem Deckblatt-Material (32), dabei insbesondere auf einer der Außenseite (30) zugewandten Oberseite des Deckblatt-Materials (32) angebracht ist.

6. Inkontinenzwegwerfwindel nach einem der vorherigen Ansprüche, **dadurch gekennzeichnet, dass** die farbige Zone (73) des Kennzeichnungsmittels (70) eine Bedruckung, insbesondere eine farbige Bedruckung ist.

7. Inkontinenzwegwerfwindel nach einem der vorherigen Ansprüche, **dadurch gekennzeichnet, dass** auf der Innenseite (28) oder dass auf der Außenseite (30), das Kennzeichnungsmittel (70) eine Fläche A1 mit einer maximalen Querstreckung E1 aufweist,
- und dass das Kennzeichnungsmittel von einem ersten imaginären Rechteck (200) gerade einbeschrieben ist, wobei das erste imaginäre Rechteck (200) erste und zweite Längsseiten (202, 203) parallel zu der Längsrichtung (4) aufweist und eine erste innere und eine äußere Querseite (204, 205) parallel zur Querrichtung (6) aufweist und wobei die erste innere Querseite (204) einen ersten Abstand D1 vom hinteren Hüftrand (12) aufweist und wobei die erste und zweite Längsseite (204, 205) eine Länge Z1 aufweisen ,
- und dass ein zweites imaginäres Rechteck (210) auf derselben Innenseite (28) oder Außenseite (30) in einem Vorderendabschnitt (24) in einer Position verortbar ist, wobei das zweite imaginäre Rechteck (210) eine dritte und eine vierte Längsseite (212, 213) parallel zu der Längsrichtung (4) aufweist, und eine zweite innere (214) und zweite äußere Querseite (215) parallel zu der Querrichtung (6) aufweist, und wobei die dritte und vierte Längsseite (212, 213) eine Länge Z1' aufweisen, die der Länge Z1 entspricht (Z1'=Z1) und wobei die zweite innere Querseite (214) einen Abstand D2 vom vorderen Hüftrand (13) aufweist, und dass für jede der Positionen, in die das zweite imaginäre Rechteck (210) verortet wird, gilt:
a) entweder innerhalb des zweiten imaginären Rechtecks (210) keine visuell erkennbare farbige Markierung (220) vorhanden ist, oder
b) im Falle einer visuell erkennbaren farbigen Markierung (220) innerhalb des zweiten imaginären Rechtecks (210) die farbige Markierung (220) eine Fläche A2 mit einem Flächenanteil von höchstens 25%, insbesondere höchstens 20%, weiter insbesondere höchstens 15%, weitere insbesondere höchstens 10 % der Fläche A1 aufweist
und/oder
c) im Falle einer visuell erkennbaren farbigen Markierung (220) innerhalb des zweiten imaginären Rechtecks (210), die farbige Markierung (220) eine maximale Querstreckung E2 aufweist, wobei die maximale Querstreckung E2 kleiner ist als die maximale Quererstreckung E1 des Kennzeichnungsmittels (70), wobei insbesondere die maximale Quererstreckung E2 höchstens 40 %, insbesondere höchstens 30% %, weiter insbesondere höchstens 20%, weitere insbesondere höchstens 10 % der maximalen Querstreckung E1 beträgt,
- wobei die Positionen, in die das zweite imaginäre Rechteck (210) verortet wird, dadurch begrenzt sind, dass das zweite imaginäre Rechteck (210) im Vergleich zum ersten imaginären Rechteck (200) parallel zur der Längsmittelachse (8) verschoben ist und für den Abstand D2 gilt:
a) D2 ≤ 1,5 x D1 und
b) die zweite äußere Querseite (215) des zweiten imaginären Rechtecks (210) sich nicht über den vorderen Hüftrand (13) hinauserstreckt.

8. Inkontinenzwegwerfwindel nach einem der vorherigen Ansprüche, **dadurch gekennzeichnet, dass** das Kennzeichnungsmittels (70) bzw. die farbige Zone (73) des ersten Kennzeichens (74) des Kennzeichnungsmittels eine maximale Quererstreckung E1 von höchstens 1/2, insbesondere höchstens 1/3, insbesondere höchstens ¼ , und insbesondere mindestens 1/6 einer Querstreckung C der Inkontinenzwegwerfwindel an einer in der Querrichtung (4) schmalsten Stelle (155) im Schrittbereich (15) aufweist.

9. Inkontinenzwegwerfwindel nach einem der vorherigen Ansprüche, **dadurch gekennzeichnet, dass** eine maximale Querstreckung E1 des Kennzeichnungsmittels (70) in Querrichtung (6) der Inkontinenzwegwerfwindel größer ist als eine maximale Längserstreckung F1 des Kennzeichnungsmittels (70) in Längsrichtung (4) der Inkontinenzwegwerfwindel.

10. Inkontinenzwegwerfwindel nach einem der vorherigen Ansprüche, **dadurch gekennzeichnet, dass** das Kennzeichnungsmittel (70) eine maximale Längserstreckung F1 aufweist und mit mindestens 50% der maximalen Längserstreckung F1, vorzugsweise mindestens 60%, weitere vorzugsweise mindestens 75%, weiter vorzugsweise mindestens 90% innerhalb eines hinteren Quernahtabschnitts (46) angeordnet ist, insbesondere bevorzugt ist das Kennzeichnungsmittel (70) vollumfänglich innerhalb eines hinteren Quernahtabschnitts (46) angeordnet.

11. Inkontinenzwegwerfwindel nach einem der vorherigen Ansprüche 2 - 10, **dadurch gekennzeichnet, dass** von einer Produkt-Fläche der Inkontinenzwegwerfwindel ein hinterer Quernahtabschnitt (46) einen Flächenanteil von 2 - 11%, bevorzugt 3 -10 %, weiter bevorzugt 3-9 % bezogen auf die Produkt-Fläche aufweist und/oder dass ein hinterer Quernahtabschnitt (46) einen Flächenanteil von 60 - 100%, bevorzugt 65-100% weiter bevorzugt 70-100% bezogen auf den Rückenendabschnitt (20) aufweist, insbesondere dass ein hinterer Quernahtabschnitt (46) eine Fläche 8.000 - 35.000 mm², vorzugsweise 10.000 - 32.000 mm², weiter vorzugsweise 12.000 -30.000 mm² aufweist.

12. Inkontinenzwegwerfwindel nach einem der vorherigen Ansprüche 2 - 11, **dadurch gekennzeichnet, dass** das Kennzeichnungsmittel (70) 25 -40%, insbesondere 30-35%, weiter insbesondere 32-34% einer Fläche des Rückenendabschnitts (20) überfängt und/ oder dass das Kennzeichnungsmittel (70) 25 -50%, vorzugsweise 27 - 48 %, weiter vorzugsweise 30 -45 %, weiter vorzugsweise 33 - 43 % einer Fläche des hinteren Quernahtabschnitts (46) überfängt.

13. Inkontinenzwegwerfwindel nach einem der vorherigen Ansprüche 1-12, **dadurch gekennzeichnet, dass** die farbige Zone (73) ausgehend von einer Randumfangslinie (78) des Kennzeichnungsmittels (70) das zweite Kennzeichen (74) derart umschließt, dass die Größe einer Fläche A3, welche von der Randumfangslinie (78) des Kennzeichnungsmittels und einer davon innerhalb und in einem konstanten ersten Abstand AB1 verlaufenden imaginären inneren Abstandslinie (110) begrenzt wird, mindestens 10%, insbesondere mindestens 15%, insbesondere mindestens 20%, insbesondere mindestens 25% einer Größe der Fläche A1 beträgt, weiter vorzugsweise die Fläche A3 höchstens 50%, weiter insbesondere höchstens 45%, weiter insbesondere höchstens 40%, weiter insbesondere höchstens 35% , weiter insbesondere höchstens 30 % einer Größe der Fläche A1 beträgt, wobei als die Fläche A1 die durch die Randumfangslinie (78) des Kennzeichnungsmittels (70) eingeschriebene Fläche definiert ist, wobei der Flächenanteil des zweiten Kennzeichens (74) an der Fläche A3 höchstens 10%, insbesondere höchstens 7%, weiter insbesondere höchstens 5%, insbesondere höchstens 2% beträgt und/oder **dadurch gekennzeichnet, dass** die Größe einer Fläche A4, welche von einer Randumfangslinie (78) des Kennzeichnungsmittels (70) und einer davon außerhalb und in einem konstanten zweiten Abstand AB2 verlaufenden imaginären Abstandslinie (112) begrenzt wird, mindestens 30%, insbesondere mindestens 40%, insbesondere mindestens 50%, insbesondere mindestens 60%, weiter insbesondere mindestens 70% der Größe einer Fläche A1 beträgt, wobei als die Fläche A1 die durch die Randumfangslinie (78) des Kennzeichnungsmittels (70) eingeschriebene Fläche definiert ist, wobei vorzugsweise mindestens 90%, insbesondere mindestens 93%, insbesondere mindestens 95% der Fläche A4 frei von weiteren farbigen Zonen, insbesondere als Beschichtungen und/oder Bedruckungen und/oder Änderungen der dem Deckblatt-Material und/oder Backsheet-Material zu eigenen optischen visuellen und/oder haptischen Beschaffenheit sind.

14. Inkontinenzwegwerfwindel nach einem der vorherigen Ansprüche 1-13, **dadurch gekennzeichnet, dass** das Kennzeichnungsmittel (70) eine durch eine Randumfangslinie (78) eingeschriebene Fläche A1 überfängt, und das zweite Kennzeichen (74) einen Flächenanteil von höchstens 50%, insbesondere höchstens 40%, weiter insbesondere höchstens 30%, weiter insbesondere höchstens 20%, und insbesondere wenigstens 3 %, insbesondere wenigstens 5 %, insbesondere wenigstens 8 % der vom Kennzeichnungsmittel überfangenen Fläche A1 einnimmt.

15. Inkontinenzwegwerfwindel nach einem der vorherigen Ansprüche 1-14, **dadurch gekennzeichnet, dass** das Kennzeichnungsmittel (70) mit einer Randumfangslinie (78) in seinen jeweils äußersten Punkten von einem imaginären Rechteck (100) tangierend eingeschrieben ist, wobei das Rechteck (100) bezüglich seiner Kanten in Längs- und Querrichtung des Inkontinenzartikels angeordnet ist und dabei sowohl in Längsrichtung als auch in Querrichtung in jeweils 10 gleich große Unterrechtecke (101) unterteilt ist, von einer Anzahl N1 an Unterrechtecken (101a), welche vollständig oder zumindest bereichsweise innerhalb der Randumfangslinie (78) lokalisiert sind und davon einer Anzahl N2 derjenigen Unterrechtecken (101b), die zumindest bereichsweise sowohl das erste Kennzeichen (72) als auch das zweite Kennzeichen (74) aufweisen, ein Verteilungsgrad von N2/N1*100% von mindestens 10 %, insbesondere mindestens 20 %, insbesondere mindestens 25%, insbesondere mindestens 30%, insbesondere mindestens 35%, weiter insbesondere von höchstens 60%, weiter insbesondere höchstens 55 %, weiter insbesondere höchstens 50%, weiter insbesondere höchstens 45% vorgesehen ist.

## Claims

1. Absorbent disposable incontinence nappy (2) having a longitudinal direction (4) and a transverse direction (6), having a longitudinal central axis (8) and a transverse central axis (10), having an inner side (28) and an outer side (30), having a main part (11) which has an absorbent body (40), a rear waist periphery (12) and a front waist periphery (13), comprising a back region (14), a front region (16), and a crotch region (15) lying therebetween in the longitudinal direction (4) which comes to rest between the legs of a user, and having rear side portions (141, 142) which are provided on both sides in the back region (14), extend at least beyond lateral central longitudinal peripheries (151, 152) of the crotch region (15) in the transverse direction (6) and each have at least one closure means (17, 18) which can be releasably fixed to the front region (16) and thus the front region (16) and the back region (14) can be connected to one another,
wherein, in the back region (14), there is provided a labelling means (70) which is arranged on the longitudinal central axis (8) and is visually recognizable at least regionally both on the inner side (28) and on the outer side (30), wherein the labelling means (70) has, in a first region (21) of the back region (14), a first label (72) which is formed by a twodimensionally extending coloured zone (73) and provides a first item of information, wherein the coloured zone (73) is visually distinguishable from a second region (22) of the back region (14) that surrounds the first label (72), **characterized in that** the labelling means (70) comprises a second label (74), which provides a second item of information, which is formed by providing cutouts (76) of the coloured zone (73) in the first label (72).

2. Disposable incontinence nappy according to Claim 1, **characterized in that** the labelling means (70) is provided in a back end portion (20), in particular is provided completely in a back end portion (20), and/or in that there is provided in the back region (14) between a contour line (42) delimiting the absorbent body (40) and the rear waist periphery (12) a rear transverse seam portion (46) in which the labelling means (70) is at least regionally arranged, preferably is completely arranged, wherein in particular the rear transverse seam portion (46) comprises or is formed from an overhang (38) of a chassis material (31)
surrounding the absorbent body (40), in particular a topsheet material (32) and/or a backsheet material (34), in a region (39) in which the chassis material (31), in particular the topsheet material (32) and/or the backsheet material (34), extends outside a contour line (42) delimiting the absorbent body (40).

3. Disposable incontinence nappy according to either of the preceding claims, **characterized in that** the labelling means (70) is an individual element.

4. Disposable incontinence nappy according to Claim 1, **characterized in that** the second label (74) comprises alphanumeric characters.

5. Disposable incontinence nappy according to any of the preceding claims, **characterized in that** the labelling means (70) is attached on a backsheet material (34), in this case on a top side of the backsheet material facing the inner side (28) or on a top side thereof facing the outer side (30), or, in particular in the case of a backsheet material comprising an inner film ply (35) and an outer nonwoven ply (36), the labelling means (70) is attached therebetween and in particular on the top side of the film ply (35) facing the outer side (30), or **in that** the labelling means (70) is attached on a topsheet material (32), in particular on a top side of the topsheet material (32) facing the outer side (30).

6. Disposable incontinence nappy according to any of the preceding claims, **characterized in that** the coloured zone (73) of the labelling means (70) is a print, in particular a coloured print.

7. Disposable incontinence nappy according to any of the preceding claims, **characterized in that**, on the inner side (28), or **in that**, on the outer side (30), the labelling means (70) has an area A1 with a maximum transverse extent E1,
- and **in that** the labelling means is specifically inscribed by a first imaginary rectangle (200), wherein the first imaginary rectangle (200) has first and second longitudinal sides (202, 203) parallel to the longitudinal direction (4) and has a first inner and an outer transverse side (204, 205) parallel to the transverse direction (6), and wherein the first inner transverse side (204) is at a first spacing D1 from the rear waist periphery (12), and wherein the first and second longitudinal sides (204, 205) have a length Z1,
- and **in that** a second imaginary rectangle (210) can be located in a position on the same inner side (28) or outer side (30) in a front end portion (24), wherein the second imaginary rectangle (210) has a third and a fourth longitudinal side (212, 213) parallel to the longitudinal direction (4) and has a second inner transverse side (214) and second outer transverse side (215) parallel to the transverse direction (6), and wherein the third and fourth longitudinal sides (212, 213) have a length Z1' which corresponds to the length Z1 (Z1'=Z1), and wherein the second inner transverse side (214) is at a spacing D2 from the front waist periphery (13), and **in that**, for each of the positions in which the second imaginary rectangle (210) is located, the following applies:
a) there is either no visually recognizable coloured marking (220) within the second imaginary rectangle (210), or
b) if there is a visually recognizable coloured marking (220) within the second imaginary rectangle (210), the coloured marking (220) has an area A2 with an areal proportion of at most 25%, in particular at most 20%, more particularly at most 15%, more particularly at most 10%, of the area A1,
and/or
c) if there is a visually recognizable coloured marking (220) within the second imaginary rectangle (210), the coloured marking (220) has a maximum transverse extent E2, wherein the maximum transverse extent E2 is smaller than the maximum transverse extent E1 of the labelling means (70), wherein in particular the maximum transverse extent E2 is at most 40%, in particular at most 30%, more particularly at most 20%, more particularly at most 10%, of the maximum transverse extent E1,
- wherein the positions in which the second imaginary rectangle (210) is located are delimited by the second imaginary rectangle (210) being displaced parallel to the longitudinal central axis (8) in comparison to the first imaginary rectangle (200), and the following applies with regard to the spacing D2:
a) D2 ≤ 1.5 x D1, and
b) the second outer transverse side (215) of the second imaginary rectangle (210) does not extend beyond the front waist periphery (13).

8. Disposable incontinence nappy according to any of the preceding claims, **characterized in that** the labelling means (70) or the coloured zone (73) of the first label (74) of the labelling means has a maximum transverse extent E1 of at most 1/2, in particular at most 1/3, in particular at most ¼, and in particular at least 1/6, of a transverse extent C of the disposable incontinence nappy at a location (155) in the crotch region (15) that is narrowest in the transverse direction (4).

9. Disposable incontinence nappy according to any of the preceding claims, **characterized in that** a maximum transverse extent E1 of the labelling means (70) in the transverse direction (6) of the disposable incontinence nappy is greater than a maximum longitudinal extent F1 of the labelling means (70) in the longitudinal direction (4) of the disposable incontinence nappy.

10. Disposable incontinence nappy according to any of the preceding claims, **characterized in that** the labelling means (70) has a maximum longitudinal extent F1 and is arranged with at least 50% of the maximum longitudinal extent F1, preferably at least 60%, more preferably at least 75%, more preferably at least 90%, within a rear transverse seam portion (46); particularly preferably, the labelling means (70) is arranged entirely within a rear transverse seam portion (46).

11. Disposable incontinence nappy according to any of the preceding Claims 2-10, **characterized in that**, based on a product area of the disposable incontinence nappy, a rear transverse seam portion (46) has an areal proportion of 2-11%, preferably 3-10%, more preferably 3-9%, of the product area, and/or **in that**, based on the back end portion (20), a rear transverse seam portion (46) has an areal proportion of 60-100%, preferably 65-100%, more preferably 70-100%, in particular **in that** a rear transverse seam portion (46) has an area of 8000-35 000 mm², preferably 10 000-32 000 mm², more preferably 12 000-30 000 mm².

12. Disposable incontinence nappy according to any of the preceding Claims 2-11, **characterized in that** the labelling means (70) overlaps 25-40%, in particular 30-35%, more particularly 32-34%, of an area of the back end portion (20), and/or **in that** the labelling means (70) overlaps 25-50%, preferably 27-48%, more preferably 30-45%, more preferably 33-43%, of an area of the rear transverse seam portion (46).

13. Disposable incontinence nappy according to any of the preceding Claims 1-12, **characterized in that** the coloured zone (73) encloses the second label (74) starting from a peripheral perimeter (78) of the labelling means (70) in such a way that the size of an area A3, which is delimited by the peripheral perimeter (78) of the labelling means and an imaginary inner spacing line (110) running within the latter and at a constant first spacing AB1 therefrom, is at least 10%, in particular at least 15%, in particular at least 20%, in particular at least 25%, of a size of the area A1, more preferably the area A3 is at most 50%, more particularly at most 45%, more particularly at most 40%, more particularly at most 35%, more particularly at most 30%, of a size of the area A1, wherein the area A1 is defined as the area inscribed by the peripheral perimeter (78) of the labelling means (70), wherein the areal proportion of the second label (74) in the area A3 is at most 10%, in particular at most 7%, more particularly at most 5%, in particular at most 2%, and/or **characterized in that** the size of an area A4, which is delimited by a peripheral perimeter (78) of the labelling means (70) and an imaginary spacing line (112) running outside the latter and at a constant second spacing AB2 therefrom, is at least 30%, in particular at least 40%, in particular at least 50%, in particular at least 60%, more particularly at least 70%, of the size of an area A1, wherein the area A1 is defined as the area inscribed by the peripheral perimeter (78) of the labelling means (70), wherein preferably at least 90%, in particular at least 93%, in particular at least 95%, of the area A4 is free from further coloured zones, in particular in the form of coatings and/or prints and/or changes in the optical, visual and/or haptic nature particular to the topsheet material and/or backsheet material.

14. Disposable incontinence nappy according to any of the preceding Claims 1-13, **characterized in that** the labelling means (70) overlaps an area A1 inscribed by a peripheral perimeter (78), and the second label (74) takes up an areal proportion of at most 50%, in particular at most 40%, more particularly at most 30%, more particularly at most 20%, and in particular at least 3%, in particular at least 5%, in particular at least 8%, of the area A1 overlapped by the labelling means.

15. Disposable incontinence nappy according to any of the preceding Claims 1-14, **characterized in that** the labelling means (70) is inscribed with a peripheral perimeter (78) at its respectively outermost points in a manner tangent to an imaginary rectangle (100), wherein the rectangle (100) is arranged with respect to its edges in the longitudinal and transverse direction of the incontinence article and is divided both in the longitudinal direction and in the transverse direction into in each case 10 same-sized sub-rectangles (101), and, of a number N1 of sub-rectangles (101a) that are located entirely or at least regionally within the peripheral perimeter (78) and a number N2 of those sub-rectangles (101b) that have at least regionally both the first label (72) and the second label (74), a degree of distribution of N2/N1*100% of at least 10%, in particular at least 20%, in particular at least 25%, in particular at least 30%, in particular at least 35%, more particularly at most 60%, more particularly at most 55%, more particularly at most 50%, more particularly at most 45%, is provided.

## Revendications

1. Couche jetable absorbante pour incontinence (2) avec un sens longitudinal (4) et un sens transversal (6), avec un axe central longitudinal (8) et un axe central transversal (10), avec un côté intérieur (28) et un côté extérieur (30), avec une partie principale (11) comportant un corps d'aspiration (40) avec un bord arrière de hanche (12) et un bord avant de hanche (13), comprenant une zone arrière (14), une zone avant (16), et une zone d'entrejambe (15) intercalée entre celles-ci dans le sens longitudinal (4), venant se poser entre les jambes d'un utilisateur, et avec des sections latérales arrière (141, 142) prévues des deux côtés dans la zone arrière (14), lesquelles s'étendent dans le sens transversal (6) au moins au-delà de bords longitudinaux (151, 152) centraux latéraux de la zone d'entrejambe (15) et comportent chacune au moins un moyen de fermeture (17, 18), qui peut être fixé de manière amovible sur la zone avant (16) et ainsi la zone avant (16) et la zone arrière (14) peuvent être reliées l'une à l'autre,
un moyen de marquage (70) disposé sur l'axe central longitudinal (8) étant prévu dans la zone arrière (14), lequel est visuellement reconnaissable au moins par endroits à la fois sur le côté intérieur (28) et sur le côté extérieur (30), le moyen de marquage (70) comportant dans une première zone (21) de la zone arrière (14) une première marque (72), qui est formée par une zone colorée (73) étendue en surface et fournit une première information, la zone colorée (73) pouvant être distinguée visuellement d'une deuxième zone (22) de la zone arrière (14) entourant la première marque (72), **caractérisée en ce que** le moyen de marquage (70) comprend une deuxième marque (74), qui fournit une deuxième information, qui est formée **en ce que** des évidements (76) de la zone colorée (73) sont prévus dans la première marque (72).

2. Couche jetable pour incontinence selon la revendication 1, **caractérisée en ce que** le moyen de marquage (70) est prévu dans une section d'extrémité arrière (20), en particulier est entièrement prévu dans une section d'extrémité arrière (20) et/ou qu'est prévue dans la zone arrière (14) entre une ligne de contour (42) délimitant le corps d'aspiration (40) et le bord arrière de hanche (12) une section de couture transversale arrière (46), dans laquelle le moyen de marquage (70) est disposé au moins par endroits, de préférence est entièrement disposé, en particulier la section de couture transversale arrière (46) comprenant un élément en porteà-faux (38) d'un matériau de châssis (31) entourant le corps d'aspiration (40), en particulier d'un matériau de feuille de couverture (32) et/ou d'un matériau de feuille de fond (34) ou en étant formée, dans une zone (39), dans laquelle le matériau de châssis (31), en particulier le matériau de feuille de couverture (32) et/ou le matériau de feuille de fond (34) s'étendent à l'extérieur d'une ligne de contour (42) délimitant le corps d'aspiration (40).

3. Couche jetable pour incontinence selon l'une des revendications précédentes, **caractérisée en ce que** le moyen de marquage (70) est un élément individuel.

4. Couche jetable pour incontinence selon la revendication 1, **caractérisée en ce que** la deuxième marque (74) comprend des caractères alphanumériques.

5. Couche jetable pour incontinence selon l'une des revendications précédentes, **caractérisée en ce que** le moyen de marquage (70) est appliqué sur le matériau de feuille de fond (34), sur un côté supérieur tourné vers le côté intérieur (28) ou sur un côté supérieur tourné vers le côté extérieur (30) du matériau de feuille de fond, ou, en particulier dans le cas d'un matériau de feuille de fond comprenant une couche intérieure de film (35) et une couche extérieure de non-tissé (36), le moyen de marquage (70) est disposé entre celles-ci et est appliqué en particulier sur le côté supérieur de la couche de film (35) tourné vers le côté extérieur (30), ou que le moyen de marquage (70) est appliqué sur un matériau de feuille de couverture (32), en particulier sur un côté supérieur du matériau de feuille de couverture (32) tournée vers le côté extérieur (30).

6. Couche jetable pour incontinence selon l'une des revendications précédentes, **caractérisée en ce que** la zone colorée (73) du moyen de marquage (70) est une impression, en particulier une impression en couleur.

7. Couche jetable pour incontinence selon l'une des revendications précédentes, **caractérisée en ce que**, sur le côté intérieur (28) ou sur le côté extérieur (30), le moyen de marquage (70) comporte une surface A1 avec une extension transversale maximale E1,
- et que le moyen de marquage est exactement inscrit dans un premier rectangle imaginaire (200), le premier rectangle imaginaire (200) comportant des premiers et deuxièmes côtés longitudinaux (202, 203) parallèlement au sens longitudinal (4) et comporte un premier côté transversal intérieur (204) et un côté transversal extérieur (205) parallèlement au sens transversal (6) et le premier côté transversal intérieur (204) présentant une première distance D1 par rapport au bord arrière de hanche (12), et les premiers et deuxièmes côtés longitudinaux (204, 205) présentant une longueur Z1,
- et qu'un deuxième rectangle imaginaire (210) peut être positionné dans une position sur le même côté intérieur (28) ou le même côté extérieur (30) dans une section d'extrémité avant (24), le deuxième rectangle imaginaire (210) comportant un troisième et un quatrième côté longitudinal (212, 213) parallèlement au sens longitudinal (4), ainsi qu'un deuxième côté transversal intérieur (214) et un deuxième côté transversal extérieur (215) parallèlement au sens transversal (6), et le troisième et le quatrième côté longitudinal (212, 213) présentant une longueur Z1', qui correspond à la longueur Z1 (Z1' = Z1), et le deuxième côté transversal intérieur (214) présentant une distance D2 par rapport au bord avant de hanche (13), et que s'applique pour chacune des positions, dans lesquelles le deuxième rectangle imaginaire (210) est positionné :
a) soit aucune marque colorée (220) visuellement reconnaissable n'est présente à l'intérieur du deuxième rectangle imaginaire (210), soit
b) dans le cas où une marque colorée (220) visuellement reconnaissable est présente à l'intérieur du deuxième rectangle imaginaire (210), la marque colorée (220) présente une surface A2 avec une proportion surfacique de 25 % au maximum, en particulier de 20 % au maximum, par ailleurs en particulier de 15 % au maximum, par ailleurs en particulier de 10 % au maximum de la surface A1.
et/ou
c) dans le cas où une marque colorée (220) visuellement reconnaissable est présente à l'intérieur du deuxième rectangle imaginaire (210), la marque colorée (220) présente une extension transversale maximale E2, l'extension transversale maximale E2 étant inférieure à l'extension transversale maximale E1 du moyen de marquage (70), l'extension transversale maximale E2 représentant en particulier 40 % au maximum, en particulier 30 % au maximum, par ailleurs en particulier 20 % au maximum, par ailleurs en particulier 10 % au maximum de l'extension transversale maximale E1,
- les positions, dans lesquelles le deuxième rectangle imaginaire (210) est positionné, étant délimitées **en ce que** le deuxième rectangle imaginaire (210), en comparaison avec le premier rectangle imaginaire (200), est coulissé parallèlement à l'axe central longitudinal (8), et s'appliquant pour la distance D2 :
a) D2 ≤ 1,5 x D1 et
b) le deuxième côté transversal extérieur (215) du deuxième rectangle imaginaire (210) ne s'étend pas au-delà du bord avant de hanche (13).

8. Couche jetable pour incontinence selon l'une des revendications précédentes, **caractérisée en ce que** le moyen de marquage (70) ou la zone colorée (73) de la première marque (74) du moyen de marquage comporte une extension transversale maximale E1 de 1/2 au maximum, en particulier de 1/3 au maximum, en particulier de ¼ au maximum, et en particulier d'au moins 1/6 d'une extension transversale C de la couche jetable pour l'incontinence sur un emplacement (155) le plus étroit dans le sens transversal (4) dans la zone d'entrejambe (15).

9. Couche jetable pour incontinence selon l'une des revendications précédentes, **caractérisée en ce qu'**une extension transversale maximale E1 du moyen de marquage (70) dans le sens transversal (6) de la couche jetable pour incontinence est supérieure à une extension longitudinale maximale F1 du moyen de marquage (70) dans le sens longitudinal (4) de la couche jetable pour incontinence.

10. Couche jetable pour incontinence selon l'une des revendications précédentes, **caractérisée en ce que** le moyen de marquage (70) comporte une extension longitudinale maximale F1 et est disposé sur au moins 50 % de l'extension longitudinale maximale F1, de préférence au moins 60 %, par ailleurs de préférence au moins 75 %, par ailleurs de préférence au moins 90 %, à l'intérieur d'une section de couture transversale arrière (46), le moyen de marquage (70) étant de manière préférée entièrement disposé à l'intérieur d'une section de couture transversale arrière (46).

11. Couche jetable pour incontinence selon l'une des revendications précédentes 2 à 10, **caractérisée en ce qu'**une section de couture transversale arrière (46) présente, d'une surface de produit de la couche jetable pour incontinence, une proportion surfacique de 2 à 11 %, de manière préférée de 3 à 10 %, de manière davantage préférée de 3 à 9 %, par rapport à la surface de produit et/ou qu'une section de couture transversale arrière (46) présente une proportion surfacique de 60 à 100 %, de manière préférée de 65 à 100 %, de manière davantage préférée de 70 à 100 %, par rapport à la section d'extrémité arrière (20), en particulier qu'une section de couture transversale arrière (46) présente une surface de 8 000 à 35 000 mm², de préférence de 10 000 à 32 000 mm², par ailleurs de préférence de 12 000 à 30 000 mm².

12. Couche jetable pour incontinence selon l'une des revendications précédentes 2 à 11, **caractérisée en ce que** le moyen de marquage (70) recouvre 25 à 40 %, en particulier 30 à 35 %, par ailleurs en particulier 32 à 34 % d'une surface de la section d'extrémité arrière (20), et/ou que le moyen de marquage (70) recouvre 25 à 50 %, de préférence 27 à 48 %, par ailleurs de préférence de 30 à 45 %, par ailleurs de préférence de 33 à 43 % d'une surface de la section de couture transversale arrière (46).

13. Couche jetable pour incontinence selon l'une des revendications précédentes 1 à 12, **caractérisée en ce que** la zone colorée (73), en partant d'une ligne de contour périphérique (78) du moyen de marquage (70), entoure la deuxième marque (74) de telle manière que la taille d'une surface A3, qui est délimitée par la ligne de contour périphérique (78) du moyen de marquage et est délimitée par une ligne de distance intérieure imaginaire (110) s'étendant à l'intérieur et à une première distance constante AB1 de celle-ci, représente au moins 10 %, en particulier au moins 15 %, en particulier au moins 20 %, en particulier au moins 25 %, d'une taille de la surface A1, la surface A3 représentant par ailleurs de préférence 50 % au maximum, par ailleurs en particulier 45 % au maximum, par ailleurs en particulier 40 % au maximum, par ailleurs en particulier 35 % au maximum, par ailleurs en particulier 30 % au maximum d'une taille de la surface A1, la surface inscrite par la ligne de contour périphérique (78) du moyen de marquage (70) étant définie comme la surface A1, la proportion surfacique de la deuxième marque (74) par rapport à la surface A3 étant de 10 % au maximum, en particulier de 7 % au maximum, par ailleurs en particulier de 5 % au maximum, en particulier de 2 % au maximum, et/ou **caractérisée en ce que** la taille d'une surface A4, qui est délimitée par une ligne de contour périphérique (78) du moyen de marquage (70) et par une ligne de distance imaginaire (112) s'étendant à l'extérieur et à une deuxième distance constante AB2 de celle-ci, représente au moins 30 %, en particulier au moins 40 %, en particulier au moins 50 %, en particulier au moins 60 %, par ailleurs en particulier au moins 70 % de la taille d'une surface A1, la surface inscrite par la ligne de contour périphérique (78) du moyen de marquage (70) étant définie comme la surface A1, de préférence au moins 90 %, en particulier au moins 93 %, en particulier au moins 95 % de la surface A4 étant de préférence exempts d'autres zones colorées, en particulier de revêtements et/ou d'impressions et/ou de modifications de la nature optique visuelle et/ou haptique propre au matériau de feuille de couverture et/ou au matériau de feuille de fond.

14. Couche jetable pour incontinence selon l'une des revendications précédentes 1 à 13, **caractérisée en ce que** le moyen de marquage (70) recouvre une surface A1 inscrite par une ligne de contour périphérique (78), et la deuxième marque (74) occupe une proportion surfacique de 50 % au maximum, en particulier de 40 % au maximum, par ailleurs en particulier de 30 % au maximum, par ailleurs en particulier de 20 % au maximum, et en particulier d'au moins 3 %, en particulier d'au moins 5 %, en particulier d'au moins 8 % de la surface A1 recouverte par le moyen de marquage.

15. Couche jetable pour incontinence selon l'une des revendications précédentes 1 à 14, **caractérisée en ce que** le moyen de marquage (70) est inscrit de manière tangente, par une ligne de contour périphérique (78), dans un rectangle imaginaire (100) sur ses points les plus extérieurs respectifs, le rectangle (100) étant disposé, par ses bords, dans le sens longitudinal et le sens transversal de l'article pour l'incontinence et étant subdivisé à la fois dans le sens longitudinal et dans le sens transversal en respectivement 10 sous-rectangles (101) de taille identique, un degré de répartition de N2/N1*100 % d'au moins 10 %, en particulier d'au moins 20 %, en particulier d'au moins 25 %, en particulier d'au moins 30 %, en particulier d'au moins 35 %, et par ailleurs en particulier de 60 % au maximum, par ailleurs en particulier de 55 % au maximum, par ailleurs en particulier de 50 % au maximum, par ailleurs en particulier de 45 % au maximum, étant prévu à partir d'un nombre N1 de sous-rectangles (101a), qui sont localisés entièrement ou au moins par endroits à l'intérieur de la ligne de contour périphérique (78), et d'un nombre N2 de ceux des sous-rectangles (101b) qui comportent au moins par endroits à la fois la première marque (72) et la deuxième marque (74).
